# EUROPEAN PATENT SPECIFICATION

(11) **EP 2 753 607 B1**
(45) Date of publication and mention of the grant of the patent: **13.04.2016**
(21) Application number: 12768941.2
(22) Date of filing: 07.09.2012
(51) Int. Cl.: C07D 239/22, C07F 9/02, G01N 33/531, G01N 33/68

(54) **BICYCLO[6.1.0]NON-4-YNE REAGENTS FOR CHEMICAL MODIFICATION OF OLIGONUCLEOTIDES**
BICYCLO[6.1.0]NON-4-YNE REAGENZIEN FÜR CHEMISCHE & xA; MODIFIZIERUNGEN VON OLIGONUKLEOTIDEN
REACTIFS BICYCLO[6.1.0]NON-4-YNE DESTINES A DES & xA; MODIFICATIONS CHIMIQUES D'OLIGONUCLÉOTIDES

(30) Priority: 09.09.2011 US 201161532867 P; 21.03.2012 US 201261613599 P
(43) Date of publication of application: 16.07.2014
(73) Proprietor: Berry And Associates, Inc., Dexter, MI 48130 (US); SynAffix B.V., 5349 AC Oss (NL)
(72) Inventor: HODGES, John, Cooke, Ann Arbor, MI 48105 (US); VAN DELFT, Floris, Louis, 6524 KZ Nijmegen (NL); VAN BERKEL, Sander, Sebastiaan, 6663 HR Lent (NL); VERKADE, Jorge, 6531 AC Nijmegen (NL); BERRY, David, A., deceased (US)
(74) Representative: D Young & Co LLP
(86) International application number: PCT/US2012/054129
(87) International publication number: WO 2013/036748

(56) References cited:
- WO-A1-2011/136645
- JAYAPRAKASH K N ET AL: "Non-Nucleoside Building Blocks for Copper-Assisted and Copper-Free Click Chemistry for the Efficient Synthesis of RNA Conjugates", ORGANIC LETTERS, AMERICAN CHEMICAL SOCIETY, US, vol. 12, no. 23, 3 December 2010 (2010-12-03), pages 5410-5413, XP002659166, ISSN: 1523-7060, DOI: 10.1021/OL102205J [retrieved on 2010-11-04]
- PIETER VAN DELFT ET AL: "Synthesis of Oligoribonucleic Acid Conjugates Using a Cyclooctyne Phosphoramidite", ORGANIC LETTERS, vol. 12, no. 23, 3 December 2010 (2010-12-03), pages 5486-5489, XP55013731, ISSN: 1523-7060, DOI: 10.1021/ol102357u
- ISAAC S. MARKS ET AL: "Strain-Promoted "Click" Chemistry for Terminal Labeling of DNA", BIOCONJUGATE CHEMISTRY, vol. 22, no. 7, 20 July 2011 (2011-07-20), pages 1259-1263, XP55013991, ISSN: 1043-1802, DOI: 10.1021/bc1003668

## Description

### BACKGROUND

Those skilled in the art of chemical synthesis of single stranded segments of nucleic acids (also known as "oligonucleotides" or "oligos") frequently wish to introduce chemical tags that facilitate the scientific study of DNA and RNA. Examples of chemical tags that are frequently employed for this purpose include fluorescent dyes, quencher dyes, luminescent compounds, biotin, desthiobiotin, antigens, enzyme cofactors, heavy isotopes, radioactive isotopes, and the like.

One highly flexible approach to introduce the chemical tag to biological macromolecules such as proteins, enzymes, antibodies, oligosaccharides, and oligonucleotides is through a two step process involving a "click" reaction (1). In the first step, an alkyne is incorporated into the macromolecule. In the second step, a chemical tag that has been modified to contain an azide group is introduced, inducing a click reaction that covalently links the macromolecule and the chemical tag via their alkyne and azide functionalities, respectively (Scheme 1). A major advantage of the click reaction approach is that a single preparation of alkyne-modified macromolecule may be divided into portions and each portion may be coupled with a different azide-modified tag. This provides an efficient means of scientific exploration of a biological macromolecule of interest since a single lot of alkyne-modified macromolecule can be tagged with a wide variety of tags using a process that is simple and offers nearly a quantitative yield.

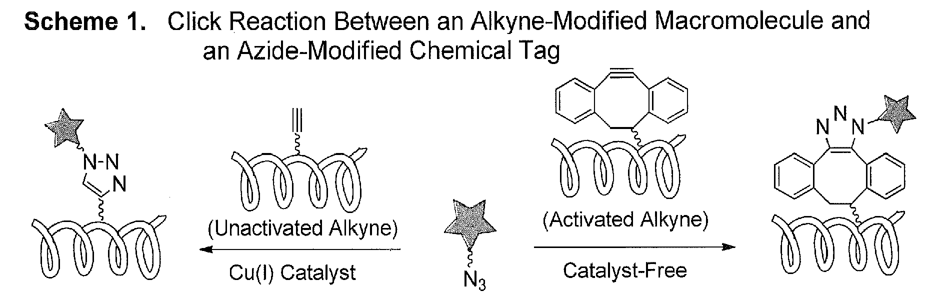

Click reactions between azide and alkyne counterparts fall into two categories (Scheme 1). The first category is the metal catalyzed click reaction, wherein a terminal alkyne and an azide react in the presence of a metal catalyst, usually a Cu(I) species (copper-catalyzed azide-alkyne cycloaddition or CuAAC). The second category is the catalyst-free click reaction, wherein an alkyne that has enhanced activity in the click reaction is used.

In the case of oligonucleotides, it is especially advantageous to employ a catalyst-free click reaction as a means of introducing chemical tags since there are numerous sites on the oligonucleotide that can coordinate with Cu(I), reducing its ability to catalyze triazole formation and causing partial degradation of the oligonucleotide (2). To this end, reagents have been developed for incorporating a dibenzocyclooctyne (DIBO) moiety into an oligonucleotide (3-5). The resulting DIBO-containing oligonucleotides undergo catalyst-free click reactions with azides. However, additional reagents for the incorporation of activated alkynes into oligonucleotides are needed. Bicyclononane (BCN) is known to undergo catalyst-free click reactions (6-7), but reagents for incorporating BCN into an oligonucleotide have not been reported.

### Background References

(1) US Patent Number 7,375,234, Copper-Catalysed Ligation of Azides and Acetylenes, May 20, 2008.
(2) Kanan, M.W.; Rozenman, M.M.; Sakurai, K.; Snyder, T.M.; Liu, D.R., Nature, 2004, 431, 545-549.
(3) Marks, I.S.; Kand, J.S.; Jones, B.T.; Landmark, K.J.; Cleland, A.J.; Taton, T.A., Bioconjugate Chemistry, 2011, 22, 1259-1263.
(4) Jayaprakash, K.N.; Peng, C.G.; Butler, D.; Varghese, J.P.; Maier, M.A.; Rajeev, K.G.; Manoharan, M., Organic Letters, 2010, 12(23), 5410-13.
(5) van Delft, P.; Meeuwenoord, N.J.; Hoogendoorn, S.; Dinkelaar, J.; Overkleeft, H.S.; van der Marel, G.A.; Filippov, D.V., Organic Letters, 2010, 12(23), 5486-5489.
(6) Dommerholt, J.; Schmidt, S.; Temming, R.; Hendriks, L.J.A.; Rutjes, F.P.J.T.; van Hest, J.C.M.; Lefeber, D.J.; Friedl, P.; van Delft, F.L., Angewande Chemie, International Edition, 2010, 49, 9422-9425.
(7) PCT/NL2011/050280, Fused Cyclooctyne Compounds and their Use in Metal-Free Click Reactions, unpublished.

### SUMMARY OF INVENTION

This invention provides phosphoramidite bicyclo[6.1.0]non-4-yne reagents and solid-supported bicyclo[6.1.0]non-4-yne reagents. These reagents are compatible with the current state of the art for the chemical synthesis of DNA oligonucleotides and RNA oligonucleotides. These reagents may be used to incorporate reactive alkyne moieties in synthetic oligonucleotides, thereby enabling further derivatization of the oligo via catalyst-free click reactions with azide-containing tags.

The first aspect of this invention is a phosphorous (III)-containing compound of Formula I: 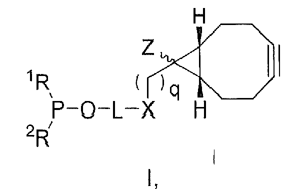 wherein:
q is 1, 2, or 3;
R¹- and R²- are independently N≡CCH₂CH₂O-, (C₁-C₆ alkyl)O-, or (C₁-C₆ alkyl)₂N-;
Z- is H-, or DMT-OCH₂-;
-X- and -L- are either both absent or both present;
-X- is absent or is -O-, -NH-, -S-, -NHCO₂-, -O₂CNH-, -NHCONH-, -NHCSNH- or -CONH-; and
-L- is absent or is selected from a group consisting of -(CH₂)ₙ-, -(CH₂CH₂O)ₙ(CH₂)ₘ-,-(CH₂CH₂CH₂O)ₙ(CH₂)ₘ-, -(CH₂)₃S₂(CH₂)₃-, -(CH₂)₆S₂(CH₂)₆-,-(CH₂)₂O(CH₂)₃S₂(CH₂)₃O(CH₂)₂-, -CH(CH₂O-DMT)CH₂-, -CH(CH₂O-DMT)CH₂O(CH₂)ₘ-, -CH(CH₂CH₂O-DMT)CH₂CH₂-, -CH(CH₂CH₂O-DMT)CH₂CH₂O(CH₂)ₘ-, 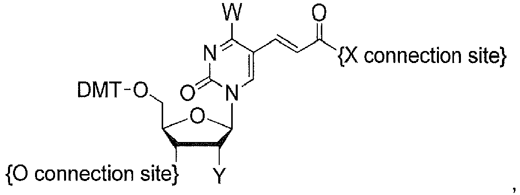 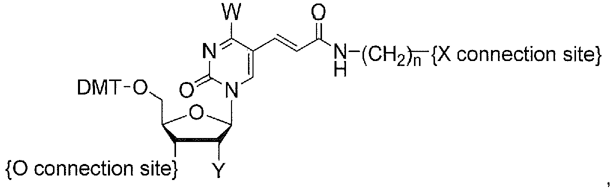 and 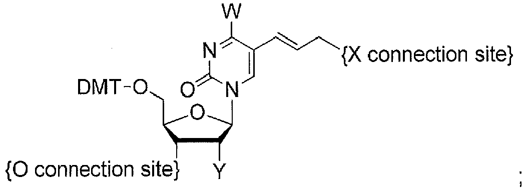
wherein n is 2-6, m is 2-3, Y is H, O-TBS, O-POM, or O-TOM, and W is OH, N=CHN(CH₃)₂, NHCOPh, or NHCOCH₃.

Any of the compounds of Formula I may be employed when a BCN group is to be installed at the 5'-terminus of an oligo. Those reagents of Formula I, wherein -L- or Z-includes a DMTO- moiety may also be employed when a BCN group is to be installed at an internal sequence position of an oligo.

### DETAILED DESCRIPTION

Wherever used within this specification, the following definitions and abbreviations apply:
"Alkyl" refers to a saturated hydrocarbon group, and includes saturated hydrocarbon groups in which the carbon atoms are arranged in a linear, branched, or cyclic fashion, or combinations thereof. "C₁-C₆ alkyl" refers to an alkyl group having one to six carbon atoms.
"BCN" is bicyclo[6.1.0]non-4-yne.
"CPG" is controlled pore glass.
"DMT" is dimethoxytrityl.
"DNA" is deoxyribonucleic acid.
"HPLC" is high performance liquid chromatography.
"Icaa" is long chain aminoalkyl, a linker that is commonly applied to controlled pore glass, the combination of which forms an insoluble solid support that is well known to those skilled in the art of DNA and RNA synthesis as Icaa-CPG.
"Mesylate" is methanesulfonate.
"MMT" is monomethoxytrityl.
"Oligo" is a shortened term for oligonucleotide.
"POM" is pivaloyloxymethyl.
"Ph" is phenyl.
"RNA" is ribonucleic acid.
"TBS" is *tert*-butyl-dimethylsilyl.
"TOM" is tri-*iso*-propylsilyloxymethyl.

### PREFERRED EMBODIMENTS OF THE COMPOUNDS OF THE INVENTION

In some embodiments, the invention includes the following compounds of Formula I:
(1) compounds wherein R¹ is N≡CCH₂CH₂O- and R² is (*i*-Pr)₂N-;
(2) compounds wherein Z is
   (a) H-; or
   (b) DMT-OCH₂-;
(3) compounds wherein -X- is
   (a) -O-, -NH-, or -NHCO₂- or is absent;
   (b) -O-, -NH-, -S-, -NHCO₂-, or-NHCONH-;
   (c) -O-, -NH-, or -S-;
   (d) -NHCO₂-, or -NHCONH-;
   (e) -NHCO₂-; or
   (f) -O-, -NH-, -S-, -NHCO₂-, -O₂CNH-, -NHCONH-, -NHCSNH-, or -CONH-, or is absent;
(4) compounds wherein -L- is
   (a) -(CH₂)ₙ-, -(CH₂CH₂O)ₙ(CH₂)ₘ-, or -(CH₂CH₂CH₂O)ₙ(CH₂)ₘ-, or is absent;
   (b) -(CH₂)₃S₂(CH₂)₃-, -(CH₂)₆S₂(CH₂)₆-, or -(CH₂)₂O(CH₂)₃S₂(CH₂)₃O(CH₂)₂-;
   (c) -(CH₂)ₙ-, -(CH₂CH₂O)ₙ(CH₂)ₘ-, -(CH₂)₃S₂(CH₂)₃-, -CH(CH₂O-DMT)CH₂O(CH₂)ₘ-, -CH(CH₂CH₂O-DMT)CH₂CH₂O(CH₂)ₘ-, 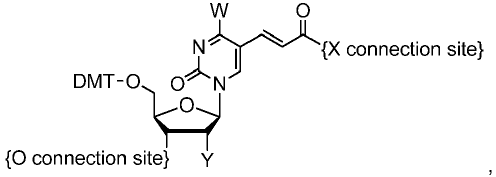 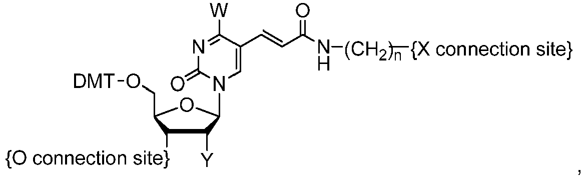 or 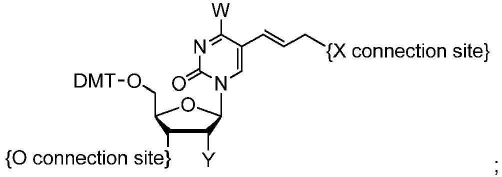
   (d) -CH(CH₂O-DMT)CH₂O(CH₂)ₘ- or -CH(CH₂CH₂O-DMT)CH₂CH₂O(CH₂)ₘ-; 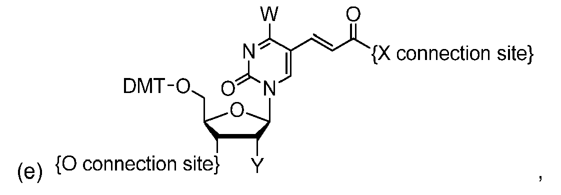 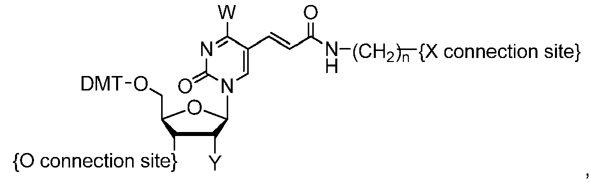 or 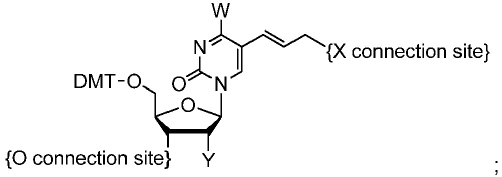 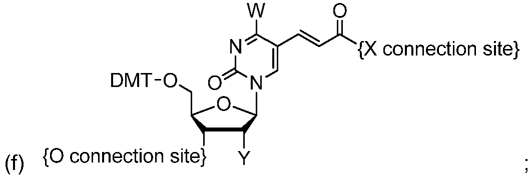 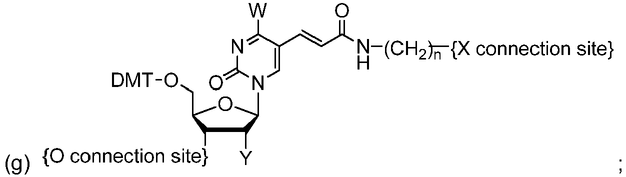 or 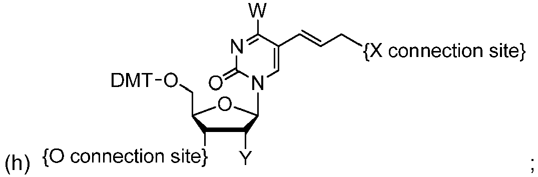
(5) compounds wherein -L- is absent or is selected from a group consisting of -(CH₂)ₙ-, -(CH₂CH₂O)ₙ(CH₂)ₘ-, -(CH₂CH₂CH₂O)ₙ(CH₂)ₘ-, -(CH₂)₃S₂(CH₂)₃-, (CH₂)₆S₂(CH₂)₆-, and -(CH₂)₂O(CH₂)₃S₂(CH₂)₃O(CH₂)₂- when Z- is DMT-OCH₂-;
(6) compounds wherein -X- and -L- are both present when q is 1;
(7) compounds wherein q is 1;
(8) compounds wherein q is 2;
(9) compounds wherein q is 3; and
(10) the compounds listed in Table 1.

It is understood that the invention also includes compounds of Formula I having any combination of the attributes listed in (1) through (9) above. For example, further embodiments of the invention can be obtained by combining (1), (2)(a), (3)(a), (4)(a), and (6); (1), (2)(a), (3)(a), (4)(b), and (6); (1), (2)(a), (3)(a), (4)(c), and (6); (1), (2)(a), (3)(a), (4)(d), and (6); (1), (2)(a), (3)(a), (4)(e), and (6); (1), (2)(a), (3)(a), (4)(f), and (6); (1), (2)(a), (3)(a), (4)(g), and (6); (1), (2)(a), (3)(a), (4)(h), and (6); (1), (2)(a), (3)(b), (4)(a), and (6); (1), (2)(a), (3)(b), (4)(b), and (6); (1), (2)(a), (3)(b), (4)(c), and (6); (1), (2)(a), (3)(b), (4)(d), and (6); (1), (2)(a), (3)(b), (4)(e), and (6); (1), (2)(a), (3)(b), (4)(f), and (6); (1), (2)(a), (3)(b), (4)(g), and (6); (1), (2)(a), (3)(b), (4)(h), and (6); (1), (2)(a), (3)(c), (4)(a), and (6); (1), (2)(a), (3)(c), (4)(b), and (6); (1), (2)(a), (3)(c), (4)(c), and (6); (1), (2)(a), (3)(c), (4)(d), and (6); (1), (2)(a), (3)(c), (4)(e), and (6); (1), (2)(a), (3)(c), (4)(f), and (6); (1), (2)(a), (3)(c), (4)(g), and (6); (1), (2)(a), (3)(c), (4)(h), and (6); (1), (2)(a), (3)(d), (4)(a), and (6); (1), (2)(a), (3)(d), (4)(b), and (6); (1), (2)(a), (3)(d), (4)(c), and (6); (1), (2)(a), (3)(d), (4)(d), and (6); (1), (2)(a), (3)(d), (4)(e), and (6); (1), (2)(a), (3)(d), (4)(f), and (6); (1), (2)(a), (3)(d), (4)(g), and (6); (1), (2)(a), (3)(d), (4)(h), and (6); (1), (2)(a), (3)(e), (4)(a), and (6); (1), (2)(a), (3)(e), (4)(b), and (6); (1), (2)(a), (3)(e), (4)(c), and (6); (1), (2)(a), (3)(e), (4)(d), and (6); (1), (2)(a), (3)(e), (4)(e), and (6); (1), (2)(a), (3)(e), (4)(f), and (6); (1), (2)(a), (3)(e), (4)(g), and (6); (1), (2)(a), (3)(e), (4)(h), and (6); (1), (2)(a), (3)(f), (4)(a), and (6); (1), (2)(a), (3)(f), (4)(b), and (6); (1), (2)(a), (3)(f), (4)(c), and (6); (1), (2)(a), (3)(f), (4)(d), and (6); (1), (2)(a), (3)(f), (4)(e), and (6); (1), (2)(a), (3)(f), (4)(f), and (6); (1), (2)(a), (3)(f), (4)(g), and (6); (1), (2)(a), (3)(f), (4)(h), and (6); (1), (2)(b), (3)(a), (4)(a), and (6); (1), (2)(b), (3)(a), (4)(b), and (6); (1), (2)(b), (3)(a), (4)(c), and (6); (1), (2)(b), (3)(a), (4)(d), and (6); (1), (2)(b), (3)(a), (4)(e), and (6); (1), (2)(b), (3)(a), (4)(f), and (6); (1), (2)(b), (3)(a), (4)(g), and (6); (1), (2)(b), (3)(a), (4)(h), and (6); (1), (2)(b), (3)(b), (4)(a), and (6); (1), (2)(b), (3)(b), (4)(b), and (6); (1), (2)(b), (3)(b), (4)(c), and (6); (1), (2)(b), (3)(b), (4)(d), and (6); (1), (2)(b), (3)(b), (4)(e), and (6); (1), (2)(b), (3)(b), (4)(f), and (6); (1), (2)(b), (3)(b), (4)(g), and (6); (1), (2)(b), (3)(b), (4)(h), and (6); (1), (2)(b), (3)(c), (4)(a), and (6); (1), (2)(b), (3)(c), (4)(b), and (6); (1), (2)(b), (3)(c), (4)(c), and (6); (1), (2)(b), (3)(c), (4)(d), and (6); (1), (2)(b), (3)(c), (4)(e), and (6); (1), (2)(b), (3)(c), (4)(f), and (6); (1), (2)(b), (3)(c), (4)(g), and (6); (1), (2)(b), (3)(c), (4)(h), and (6); (1), (2)(b), (3)(d), (4)(a), and (6); (1), (2)(b), (3)(d), (4)(b), and (6); (1), (2)(b), (3)(d), (4)(c), and (6); (1), (2)(b), (3)(d), (4)(d), and (6); (1), (2)(b), (3)(d), (4)(e), and (6); (1), (2)(b), (3)(d), (4)(f), and (6); (1), (2)(b), (3)(d), (4)(g), and (6); (1), (2)(b), (3)(d), (4)(h), and (6); (1), (2)(b), (3)(e), (4)(a), and (6); (1), (2)(b), (3)(e), (4)(b), and (6); (1), (2)(b), (3)(e), (4)(c), and (6); (1), (2)(b), (3)(e), (4)(d), and (6); (1), (2)(b), (3)(e), (4)(e), and (6); (1), (2)(b), (3)(e), (4)(f), and (6); (1), (2)(b), (3)(e), (4)(g), and (6); (1), (2)(b), (3)(e), (4)(h), and (6); (1), (2)(b), (3)(f), (4)(a), and (6); (1), (2)(b), (3)(f), (4)(b), and (6); (1), (2)(b), (3)(f), (4)(c), and (6); (1), (2)(b), (3)(f), (4)(d), and (6); (1), (2)(b), (3)(f), (4)(e), and (6); (1), (2)(b), (3)(f), (4)(f), and (6); (1), (2)(b), (3)(f), (4)(g), and (6); (1), (2)(b), (3)(f), (4)(h), and (6); (5) and (6); (1), (2)(a), (3)(a), (4)(a), and (7); (1), (2)(a), (3)(a), (4)(b), and (7); (1), (2)(a), (3)(a), (4)(c), and (7); (1), (2)(a), (3)(a), (4)(d), and (7); (1), (2)(a), (3)(a), (4)(e), and (7); (1), (2)(a), (3)(a), (4)(f), and (7); (1), (2)(a), (3)(a), (4)(g), and (7); (1), (2)(a), (3)(a), (4)(h), and (7); (1), (2)(a), (3)(b), (4)(a), and (7); (1), (2)(a), (3)(b), (4)(b), and (7); (1), (2)(a), (3)(b), (4)(c), and (7); (1), (2)(a), (3)(b), (4)(d), and (7); (1), (2)(a), (3)(b), (4)(e), and (7); (1), (2)(a), (3)(b), (4)(f), and (7); (1), (2)(a), (3)(b), (4)(g), and (7); (1), (2)(a), (3)(b), (4)(h), and (7); (1), (2)(a), (3)(c), (4)(a), and (7); (1), (2)(a), (3)(c), (4)(b), and (7); (1), (2)(a), (3)(c), (4)(c), and (7); (1), (2)(a), (3)(c), (4)(d), and (7); (1), (2)(a), (3)(c), (4)(e), and (7); (1), (2)(a), (3)(c), (4)(f), and (7); (1), (2)(a), (3)(c), (4)(g), and (7); (1), (2)(a), (3)(c), (4)(h), and (7); (1), (2)(a), (3)(d), (4)(a), and (7); (1), (2)(a), (3)(d), (4)(b), and (7); (1), (2)(a), (3)(d), (4)(c), and (7); (1), (2)(a), (3)(d), (4)(d), and (7); (1), (2)(a), (3)(d), (4)(e), and (7); (1), (2)(a), (3)(d), (4)(f), and (7); (1), (2)(a), (3)(d), (4)(g), and (7); (1), (2)(a), (3)(d), (4)(h), and (7); (1), (2)(a), (3)(e), (4)(a), and (7); (1), (2)(a), (3)(e), (4)(b), and (7); (1), (2)(a), (3)(e), (4)(c), and (7); (1), (2)(a), (3)(e), (4)(d), and (7); (1), (2)(a), (3)(e), (4)(e), and (7); (1), (2)(a), (3)(e), (4)(f), and (7); (1), (2)(a), (3)(e), (4)(g), and (7); (1), (2)(a), (3)(e), (4)(h), and (7); (1), (2)(a), (3)(f), (4)(a), and (7); (1), (2)(a), (3)(f), (4)(b), and (7); (1), (2)(a), (3)(f), (4)(c), and (7); (1), (2)(a), (3)(f), (4)(d), and (7); (1), (2)(a), (3)(f), (4)(e), and (7); (1), (2)(a), (3)(f), (4)(f), and (7); (1), (2)(a), (3)(f), (4)(g), and (7); (1), (2)(a), (3)(f), (4)(h), and (7); (1), (2)(b), (3)(a), (4)(a), and (7); (1), (2)(b), (3)(a), (4)(b), and (7); (1), (2)(b), (3)(a), (4)(c), and (7); (1), (2)(b), (3)(a), (4)(d), and (7); (1), (2)(b), (3)(a), (4)(e), and (7); (1), (2)(b), (3)(a), (4)(f), and (7); (1), (2)(b), (3)(a), (4)(g), and (7); (1), (2)(b), (3)(a), (4)(h), and (7); (1), (2)(b), (3)(b), (4)(a), and (7); (1), (2)(b), (3)(b), (4)(b), and (7); (1), (2)(b), (3)(b), (4)(c), and (7); (1), (2)(b), (3)(b), (4)(d), and (7); (1), (2)(b), (3)(b), (4)(e), and (7); (1), (2)(b), (3)(b), (4)(f), and (7); (1), (2)(b), (3)(b), (4)(g), and (7); (1), (2)(b), (3)(b), (4)(h), and (7); (1), (2)(b), (3)(c), (4)(a), and (7); (1), (2)(b), (3)(c), (4)(b), and (7); (1), (2)(b), (3)(c), (4)(c), and (7); (1), (2)(b), (3)(c), (4)(d), and (7); (1), (2)(b), (3)(c), (4)(e), and (7); (1), (2)(b), (3)(c), (4)(f), and (7); (1), (2)(b), (3)(c), (4)(g), and (7); (1), (2)(b), (3)(c), (4)(h), and (7); (1), (2)(b), (3)(d), (4)(a), and (7); (1), (2)(b), (3)(d), (4)(b), and (7); (1), (2)(b), (3)(d), (4)(c), and (7); (1), (2)(b), (3)(d), (4)(d), and (7); (1), (2)(b), (3)(d), (4)(e), and (7); (1), (2)(b), (3)(d), (4)(f), and (7); (1), (2)(b), (3)(d), (4)(g), and (7); (1), (2)(b), (3)(d), (4)(h), and (7); (1), (2)(b), (3)(e), (4)(a), and (7); (1), (2)(b), (3)(e), (4)(b), and (7); (1), (2)(b), (3)(e), (4)(c), and (7); (1), (2)(b), (3)(e), (4)(d), and (7); (1), (2)(b), (3)(e), (4)(e), and (7); (1), (2)(b), (3)(e), (4)(f), and (7); (1), (2)(b), (3)(e), (4)(g), and (7); (1), (2)(b), (3)(e), (4)(h), and (7); (1), (2)(b), (3)(f), (4)(a), and (7); (1), (2)(b), (3)(f), (4)(b), and (7); (1), (2)(b), (3)(f), (4)(c), and (7); (1), (2)(b), (3)(f), (4)(d), and (7); (1), (2)(b), (3)(f), (4)(e), and (7); (1), (2)(b), (3)(f), (4)(f), and (7); (1), (2)(b), (3)(f), (4)(g), and (7); (1), (2)(b), (3)(f), (4)(h), and (7); (5) and (7); (6) and (7); (1), (2)(a), (3)(a), (4)(a), and (8); (1), (2)(a), (3)(a), (4)(b), and (8); (1), (2)(a), (3)(a), (4)(c), and (8); (1), (2)(a), (3)(a), (4)(d), and (8); (1), (2)(a), (3)(a), (4)(e), and (8); (1), (2)(a), (3)(a), (4)(f), and (8); (1), (2)(a), (3)(a), (4)(g), and (8); (1), (2)(a), (3)(a), (4)(h), and (8); (1), (2)(a), (3)(b), (4)(a), and (8); (1), (2)(a), (3)(b), (4)(b), and (8); (1), (2)(a), (3)(b), (4)(c), and (8); (1), (2)(a), (3)(b), (4)(d), and (8); (1), (2)(a), (3)(b), (4)(e), and (8); (1), (2)(a), (3)(b), (4)(f), and (8); (1), (2)(a), (3)(b), (4)(g), and (8); (1), (2)(a), (3)(b), (4)(h), and (8); (1), (2)(a), (3)(c), (4)(a), and (8); (1), (2)(a), (3)(c), (4)(b), and (8); (1), (2)(a), (3)(c), (4)(c), and (8); (1), (2)(a), (3)(c), (4)(d), and (8); (1), (2)(a), (3)(c), (4)(e), and (8); (1), (2)(a), (3)(c), (4)(f), and (8); (1), (2)(a), (3)(c), (4)(g), and (8); (1), (2)(a), (3)(c), (4)(h), and (8); (1), (2)(a), (3)(d), (4)(a), and (8); (1), (2)(a), (3)(d), (4)(b), and (8); (1), (2)(a), (3)(d), (4)(c), and (8); (1), (2)(a), (3)(d), (4)(d), and (8); (1), (2)(a), (3)(d), (4)(e), and (8); (1), (2)(a), (3)(d), (4)(f), and (8); (1), (2)(a), (3)(d), (4)(g), and (8); (1), (2)(a), (3)(d), (4)(h), and (8); (1), (2)(a), (3)(e), (4)(a), and (8); (1), (2)(a), (3)(e), (4)(b), and (8); (1), (2)(a), (3)(e), (4)(c), and (8); (1), (2)(a), (3)(e), (4)(d), and (8); (1), (2)(a), (3)(e), (4)(e), and (8); (1), (2)(a), (3)(e), (4)(f), and (8); (1), (2)(a), (3)(e), (4)(g), and (8); (1), (2)(a), (3)(e), (4)(h), and (8); (1), (2)(a), (3)(f), (4)(a), and (8); (1), (2)(a), (3)(f), (4)(b), and (8); (1), (2)(a), (3)(f), (4)(c), and (8); (1), (2)(a), (3)(f), (4)(d), and (8); (1), (2)(a), (3)(f), (4)(e), and (8); (1), (2)(a), (3)(f), (4)(f), and (8); (1), (2)(a), (3)(f), (4)(g), and (8); (1), (2)(a), (3)(f), (4)(h), and (8); (1), (2)(b), (3)(a), (4)(a), and (8); (1), (2)(b), (3)(a), (4)(b), and (8); (1), (2)(b), (3)(a), (4)(c), and (8); (1), (2)(b), (3)(a), (4)(d), and (8); (1), (2)(b), (3)(a), (4)(e), and (8); (1), (2)(b), (3)(a), (4)(f), and (8); (1), (2)(b), (3)(a), (4)(g), and (8); (1), (2)(b), (3)(a), (4)(h), and (8); (1), (2)(b), (3)(b), (4)(a), and (8); (1), (2)(b), (3)(b), (4)(b), and (8); (1), (2)(b), (3)(b), (4)(c), and (8); (1), (2)(b), (3)(b), (4)(d), and (8); (1), (2)(b), (3)(b), (4)(e), and (8); (1), (2)(b), (3)(b), (4)(f), and (8); (1), (2)(b), (3)(b), (4)(g), and (8); (1), (2)(b), (3)(b), (4)(h), and (8); (1), (2)(b), (3)(c), (4)(a), and (8); (1), (2)(b), (3)(c), (4)(b), and (8); (1), (2)(b), (3)(c), (4)(c), and (8); (1), (2)(b), (3)(c), (4)(d), and (8); (1), (2)(b), (3)(c), (4)(e), and (8); (1), (2)(b), (3)(c), (4)(f), and (8); (1), (2)(b), (3)(c), (4)(g), and (8); (1), (2)(b), (3)(c), (4)(h), and (8); (1), (2)(b), (3)(d), (4)(a), and (8); (1), (2)(b), (3)(d), (4)(b), and (8); (1), (2)(b), (3)(d), (4)(c), and (8); (1), (2)(b), (3)(d), (4)(d), and (8); (1), (2)(b), (3)(d), (4)(e), and (8); (1), (2)(b), (3)(d), (4)(f), and (8); (1), (2)(b), (3)(d), (4)(g), and (8); (1), (2)(b), (3)(d), (4)(h), and (8); (1), (2)(b), (3)(e), (4)(a), and (8); (1), (2)(b), (3)(e), (4)(b), and (8); (1), (2)(b), (3)(e), (4)(c), and (8); (1), (2)(b), (3)(e), (4)(d), and (8); (1), (2)(b), (3)(e), (4)(e), and (8); (1), (2)(b), (3)(e), (4)(f), and (8); (1), (2)(b), (3)(e), (4)(g), and (8); (1), (2)(b), (3)(e), (4)(h), and (8); (1), (2)(b), (3)(f), (4)(a), and (8); (1), (2)(b), (3)(f), (4)(b), and (8); (1), (2)(b), (3)(f), (4)(c), and (8); (1), (2)(b), (3)(f), (4)(d), and (8); (1), (2)(b), (3)(f), (4)(e), and (8); (1), (2)(b), (3)(f), (4)(f), and (8); (1), (2)(b), (3)(f), (4)(g), and (8); (1), (2)(b), (3)(f), (4)(h), and (8); (5) and (8); (6) and (8); (1), (2)(a), (3)(a), (4)(a), and (9); (1), (2)(a), (3)(a), (4)(b), and (9); (1), (2)(a), (3)(a), (4)(c), and (9); (1), (2)(a), (3)(a), (4)(d), and (9); (1), (2)(a), (3)(a), (4)(e), and (9); (1), (2)(a), (3)(a), (4)(f), and (9); (1), (2)(a), (3)(a), (4)(g), and (9); (1), (2)(a), (3)(a), (4)(h), and (9); (1), (2)(a), (3)(b), (4)(a), and (9); (1), (2)(a), (3)(b), (4)(b), and (9); (1), (2)(a), (3)(b), (4)(c), and (9); (1), (2)(a), (3)(b), (4)(d), and (9); (1), (2)(a), (3)(b), (4)(e), and (9); (1), (2)(a), (3)(b), (4)(f), and (9); (1), (2)(a), (3)(b), (4)(g), and (9); (1), (2)(a), (3)(b), (4)(h), and (9); (1), (2)(a), (3)(c), (4)(a), and (9); (1), (2)(a), (3)(c), (4)(b), and (9); (1), (2)(a), (3)(c), (4)(c), and (9); (1), (2)(a), (3)(c), (4)(d), and (9); (1), (2)(a), (3)(c), (4)(e), and (9); (1), (2)(a), (3)(c), (4)(f), and (9); (1), (2)(a), (3)(c), (4)(g), and (9); (1), (2)(a), (3)(c), (4)(h), and (9); (1), (2)(a), (3)(d), (4)(a), and (9); (1), (2)(a), (3)(d), (4)(b), and (9); (1), (2)(a), (3)(d), (4)(c), and (9); (1), (2)(a), (3)(d), (4)(d), and (9); (1), (2)(a), (3)(d), (4)(e), and (9); (1), (2)(a), (3)(d), (4)(f), and (9); (1), (2)(a), (3)(d), (4)(g), and (9); (1), (2)(a), (3)(d), (4)(h), and (9); (1), (2)(a), (3)(e), (4)(a), and (9); (1), (2)(a), (3)(e), (4)(b), and (9); (1), (2)(a), (3)(e), (4)(c), and (9); (1), (2)(a), (3)(e), (4)(d), and (9); (1), (2)(a), (3)(e), (4)(e), and (9); (1), (2)(a), (3)(e), (4)(f), and (9); (1), (2)(a), (3)(e), (4)(g), and (9); (1), (2)(a), (3)(e), (4)(h), and (9); (1), (2)(a), (3)(f), (4)(a), and (9); (1), (2)(a), (3)(f), (4)(b), and (9); (1), (2)(a), (3)(f), (4)(c), and (9); (1), (2)(a), (3)(f), (4)(d), and (9); (1), (2)(a), (3)(f), (4)(e), and (9); (1), (2)(a), (3)(f), (4)(f), and (9); (1), (2)(a), (3)(f), (4)(g), and (9); (1), (2)(a), (3)(f), (4)(h), and (9); (1), (2)(b), (3)(a), (4)(a), and (9); (1), (2)(b), (3)(a), (4)(b), and (9); (1), (2)(b), (3)(a), (4)(c), and (9); (1), (2)(b), (3)(a), (4)(d), and (9); (1), (2)(b), (3)(a), (4)(e), and (9); (1), (2)(b), (3)(a), (4)(f), and (9); (1), (2)(b), (3)(a), (4)(g), and (9); (1), (2)(b), (3)(a), (4)(h), and (9); (1), (2)(b), (3)(b), (4)(a), and (9); (1), (2)(b), (3)(b), (4)(b), and (9); (1), (2)(b), (3)(b), (4)(c), and (9); (1), (2)(b), (3)(b), (4)(d), and (9); (1), (2)(b), (3)(b), (4)(e), and (9); (1), (2)(b), (3)(b), (4)(f), and (9); (1), (2)(b), (3)(b), (4)(g), and (9); (1), (2)(b), (3)(b), (4)(h), and (9); (1), (2)(b), (3)(c), (4)(a), and (9); (1), (2)(b), (3)(c), (4)(b), and (9); (1), (2)(b), (3)(c), (4)(c), and (9); (1), (2)(b), (3)(c), (4)(d), and (9); (1), (2)(b), (3)(c), (4)(e), and (9); (1), (2)(b), (3)(c), (4)(f), and (9); (1), (2)(b), (3)(c), (4)(g), and (9); (1), (2)(b), (3)(c), (4)(h), and (9); (1), (2)(b), (3)(d), (4)(a), and (9); (1), (2)(b), (3)(d), (4)(b), and (9); (1), (2)(b), (3)(d), (4)(c), and (9); (1), (2)(b), (3)(d), (4)(d), and (9); (1), (2)(b), (3)(d), (4)(e), and (9); (1), (2)(b), (3)(d), (4)(f), and (9); (1), (2)(b), (3)(d), (4)(g), and (9); (1), (2)(b), (3)(d), (4)(h), and (9); (1), (2)(b), (3)(e), (4)(a), and (9); (1), (2)(b), (3)(e), (4)(b), and (9); (1), (2)(b), (3)(e), (4)(c), and (9); (1), (2)(b), (3)(e), (4)(d), and (9); (1), (2)(b), (3)(e), (4)(e), and (9); (1), (2)(b), (3)(e), (4)(f), and (9); (1), (2)(b), (3)(e), (4)(g), and (9); (1), (2)(b), (3)(e), (4)(h), and (9); (1), (2)(b), (3)(f), (4)(a), and (9); (1), (2)(b), (3)(f), (4)(b), and (9); (1), (2)(b), (3)(f), (4)(c), and (9); (1), (2)(b), (3)(f), (4)(d), and (9); (1), (2)(b), (3)(f), (4)(e), and (9); (1), (2)(b), (3)(f), (4)(f), and (9); (1), (2)(b), (3)(f), (4)(g), and (9); (1), (2)(b), (3)(f), (4)(h), and (9); (5) and (9); (6) and (9); and the like.

As is readily apparent to those skilled in the art, the compounds of formula I may exist in more than one tautomeric form known as "tautomers." For example, thymine may exist as either the 5-methylpyrimidine-2,4(1H,3H)-dione tautomer or the 4-hydroxy-5-methylpyrimidin-2(1H)-one tautomer. Where tautomers exist, each tautomeric form, and mixtures thereof, are contemplated as included in the present invention. When any reference in this application to one of the specific tautomers of the compounds of formula I is given, it is understood to encompass every tautomeric form and mixtures thereof.

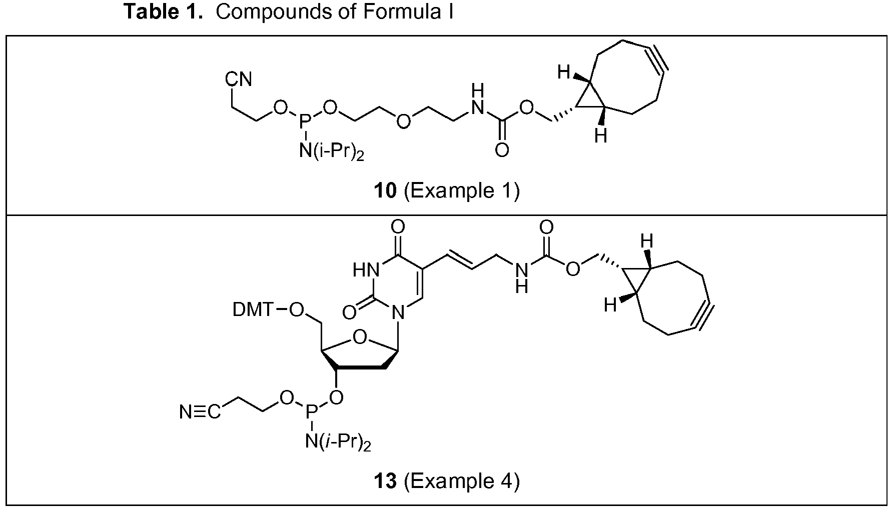

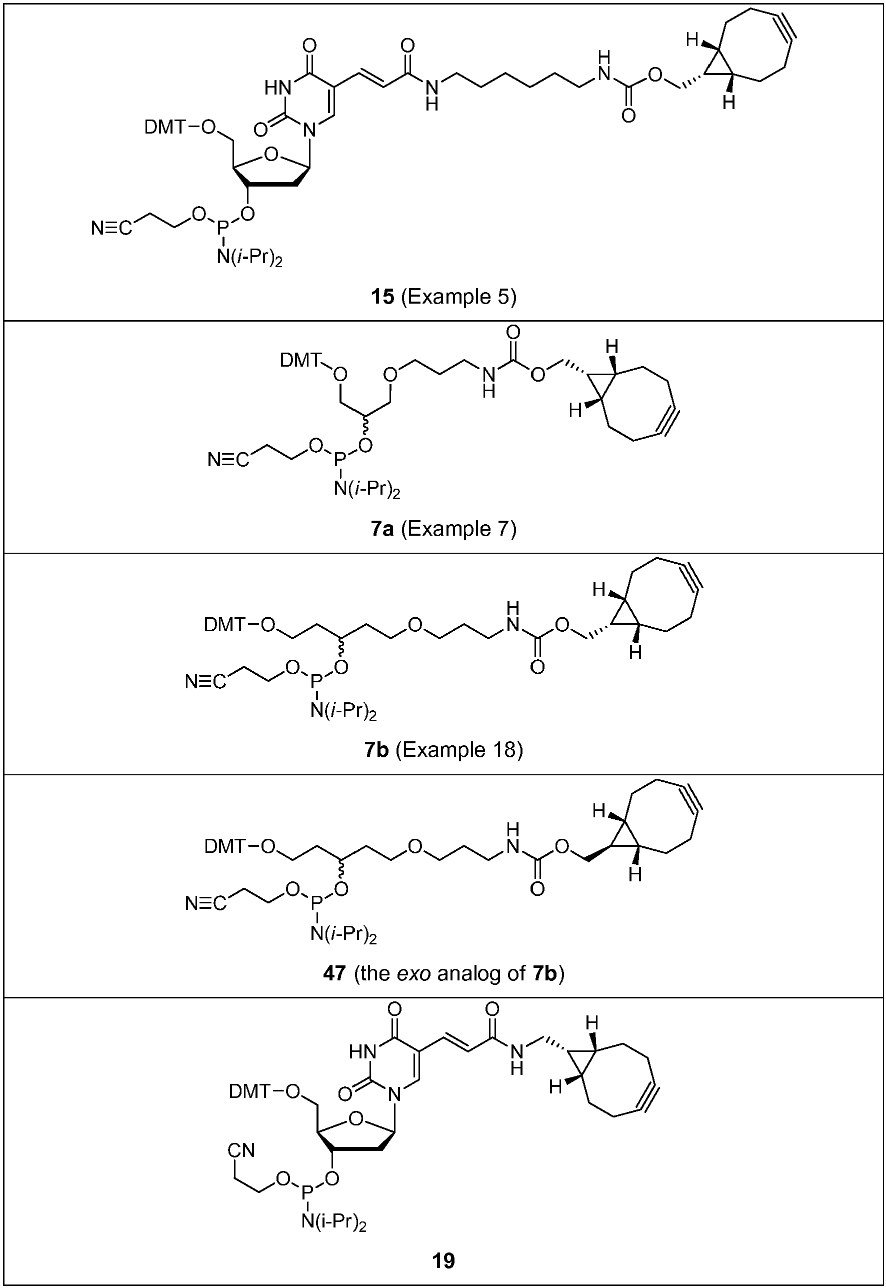

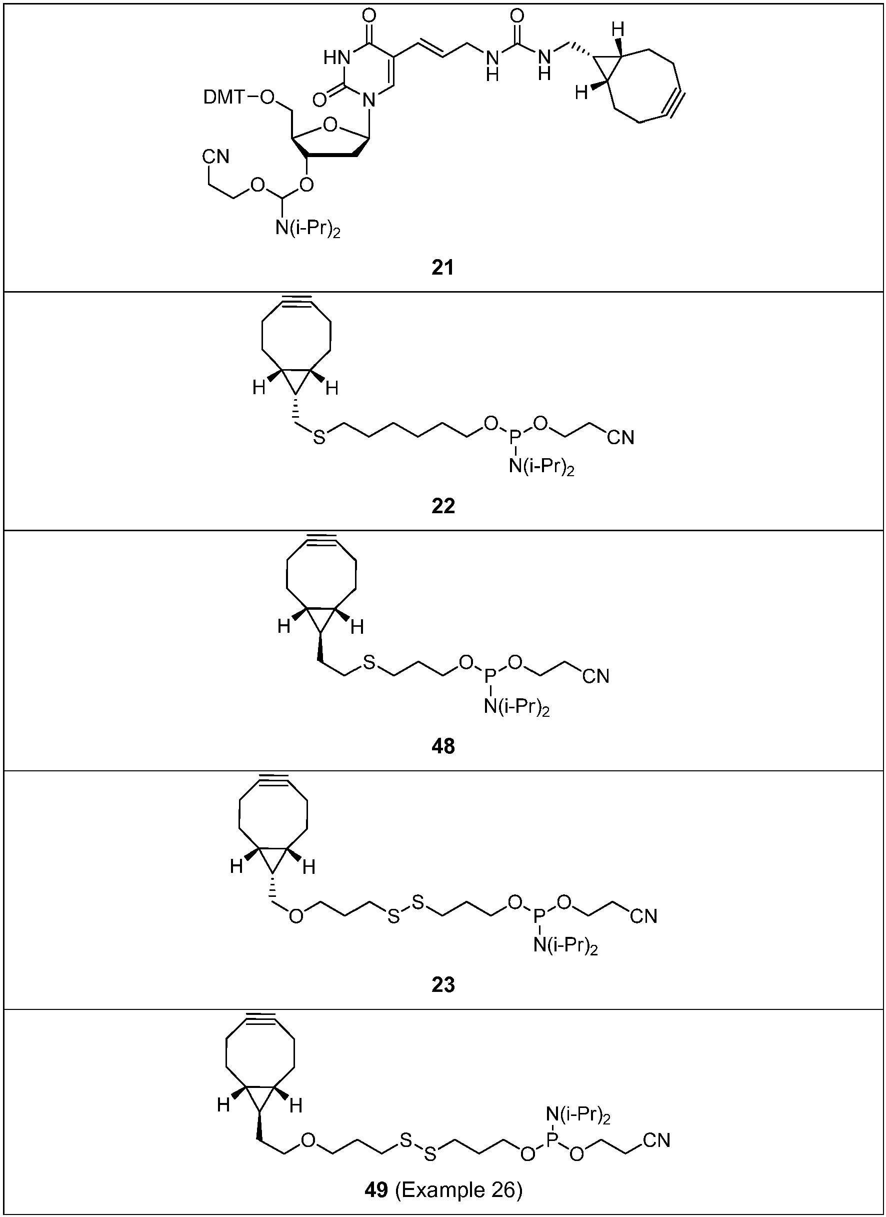

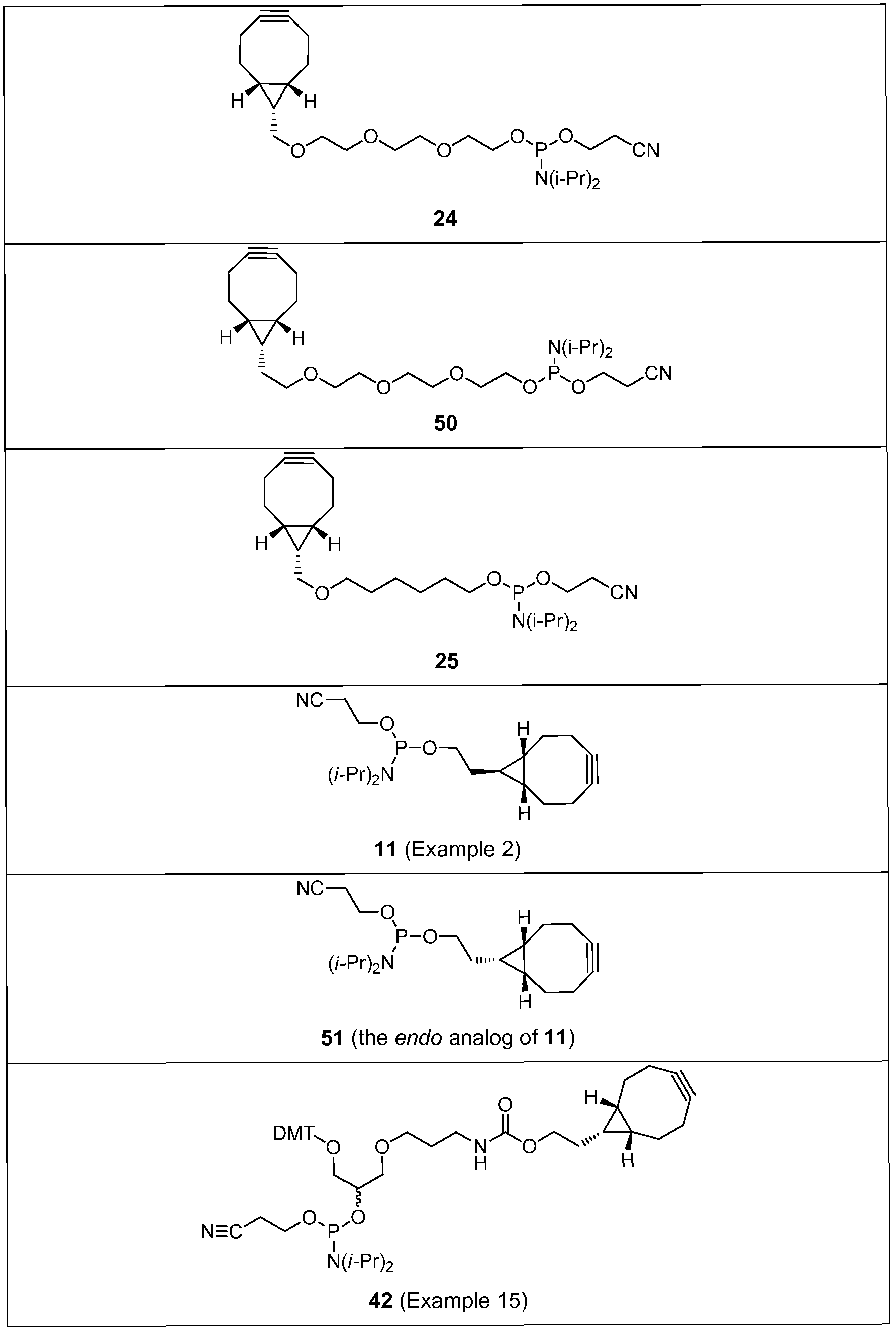

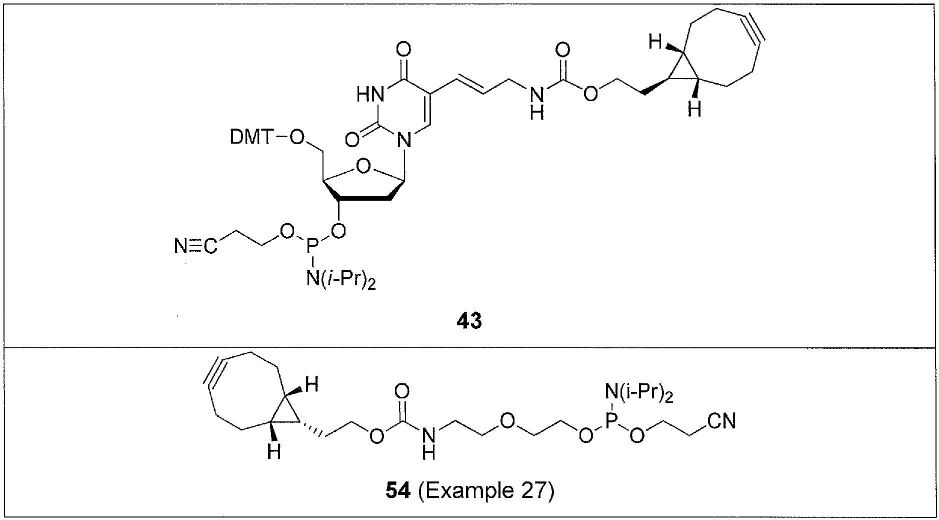

### METHODS OF PREPARATION OF COMPOUNDS OF FORMULA I

The compounds of Formula **I** may be prepared from known compounds using synthetic transformations familiar to those having ordinary skill in the art. More specifically, each of the compounds of Formula I may be prepared from BCN-methanol (***vi(a)***)*,* DMT-OCH₂-BCN-methanol (***vi(b)***)*,* BCN-ethanol (***vi(c)***)*,* BCN-propanol (***vi(d)***)*,* DMT-OCH₂-BCN-ethanol (***vi(e)***)*,* or DMT-OCH₂-BCN-propanol (***vi(f)***)*,* which all may be prepared from 1,5-cyclooctadiene as shown in Scheme 2.

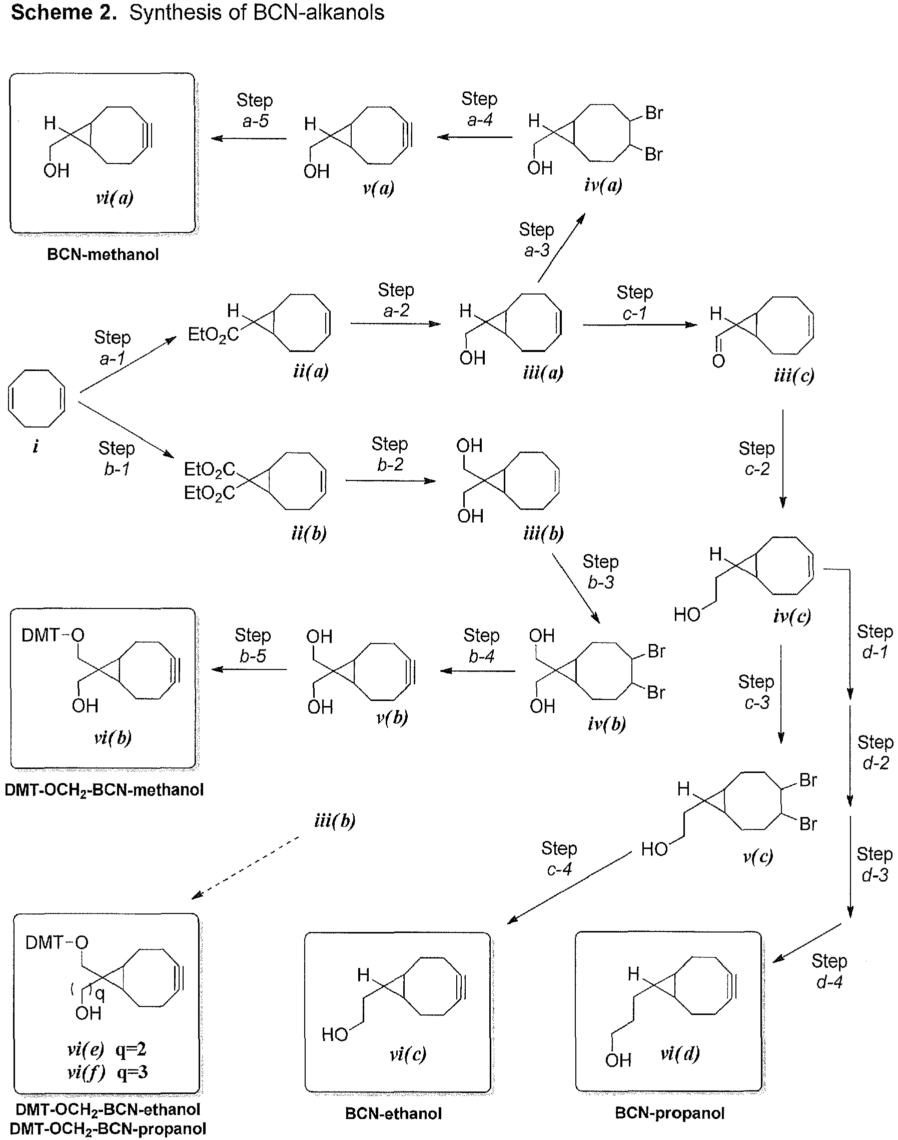

The synthesis of ***vi(a)*** from 1,5-cyclooctadiene, as illustrated in Scheme 2, has been described previously by Dommerholt et al., Angewandte Chemie, International Edition, 2010, 49, 9422-9425. The synthesis of ***vi(b)*** proceeds in analogous fashion. The syntheses of ***vi(c)*** and *vi(d)* proceed via single application (Steps *c*-*1* and *c-2*) or double application (Steps *c-1* and *c-2* followed by Steps *d-1* and *d-2*) of the Wittig one-carbon homologation method. Subsequent conversion of the cyclooctenes to cyclooctynes is achieved through analogous bromination and dehydrobromination reactions. Detailed procedures for the syntheses of ***vi(b-d)*** are found in the Examples below. It is recognized by those with ordinary skill in the art of organic synthesis that DMT protection of one of the alcohol groups in ***iii(b)*** affords a suitable synthetic intermediate that may likewise undergo one or two Wittig one-carbon homologation(s) and analogous transformation of the ring double bond to a triple bond to provide DMT-OCH₂-BCN-ethanol (***vi(e)***) and DMT-OCH₂-BCN-propanol ***(vi(f)).***

While both known diastereomers of BCN-methanol, the exo and *endo* isomers (Scheme 3), readily undergo Cu-free click reactions at room temperature, the reaction of the *endo* isomer of BCN-methanol is slightly faster than that of the exo isomer of BCN-methanol. Thus, the *endo* isomer of compounds of Formula **I** wherein q is 1 may be preferred for applications where a fast reaction rate is vital. With BCN-ethanol and BCN-propanol, both *exo* and *endo* isomers undergo Cu-free click reactions at comparable rates. In the practice of chemical tagging of synthetic oligonucleotides, both *endo* and *exo* isomers of all compounds of Formula I are generally sufficiently reactive to be useful. For simplicity of experimental design and ease of interpretation of results, it is preferable to choose one or the other and not work with a mixture of both isomers, however the use of a mixture of *endo-* and *exo*- isomers is chemically feasible.

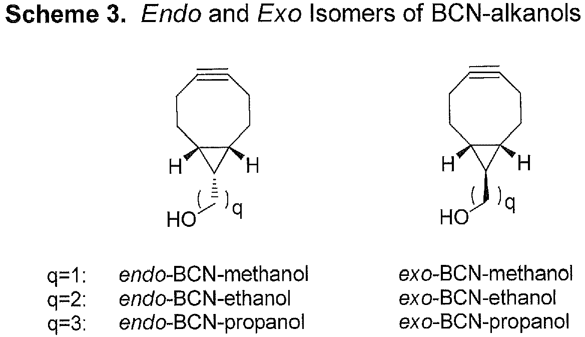

The compounds of Formula **I** may be prepared from ***vi(a-f)*** according to the methods illustrated in Schemes 4a, 4b and 4c. The procedures set forth in these schemes may be carried out with the *endo* isomer and/or the *exo* isomer of ***vi(a-f).*** Therefore, and for the sake of clarity, the specific isomers of ***vi(a-f),*** subsequent synthetic intermediates, and compounds of Formulae **I** are not specified in the schemes.

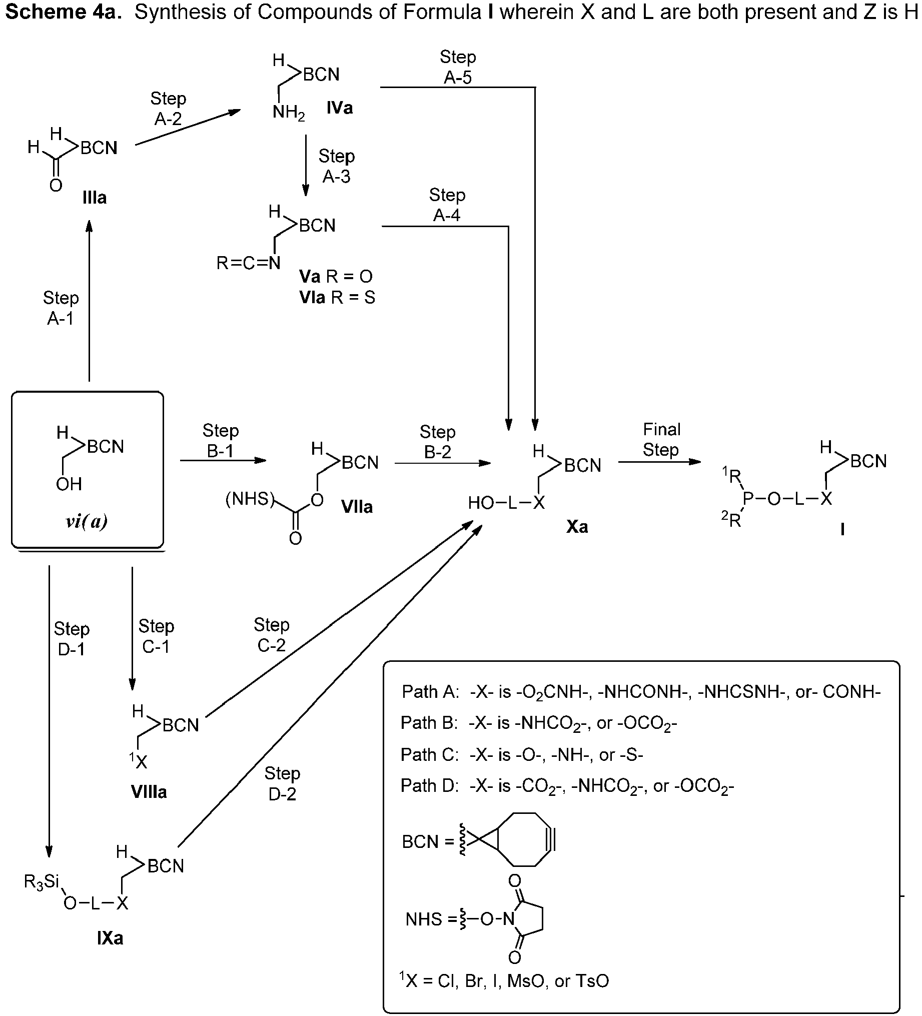

Scheme 4a illustrates the synthesis of the compounds of Formula **Xa** when X and L are both present, and Z is H. When -X- is -O₂CNH-, -NHCONH-, -NHCSNH-, or - CONH-, the compounds of Formula **Xa** may be prepared from BCN-methanol **(*vi(a)*)** in three or four steps via Path A. In Step A-1, ***vi(a)*** is converted to the aldehyde, **IIIa,** using oxidation procedures known to those with ordinary skill in the art of organic synthesis, such as Swern oxidation, Dess-Martin oxidation, Ley oxidation, chromium-based oxidations, and the like. Subsequently in Step A-2, treatment of **IIIa** with ammonia and a hydride reducing agent such as NaCNBH₃, NaBH₄, or Na(OAc)₃H, gives the amine, **IVa.** This amine is a versatile synthetic intermediate, which may be converted in Step A-3 to the isocyanate, **Va,** by treatment with phosgene, or a phosgene equivalent, such as triphosgene, phenyl-chloroformate, 4-nitrophenyl-chloroformate, di-(2-pyridyl)-carbonate, or carbonyl-diimidazole, and a tertiary amine, such as N-methylmorpholine, triethylamine, or diisopropylethylamine. Similarly, **IVa** may be converted in Step A-3 to the isothiocyanate, **VIa,** by treatment with thiophosgene, or a thiophosgene equivalent such as thiocarbonyldiimidazole or di-(2-pyridyl)-thiocarbonate, and a tertiary amine such as N-methylmorpholine, triethylamine, or diisopropylethylamine. In Step A-4, treatment of **Va** or **VIa** with a primary amine affords compounds of formula **Xa** wherein -X- is -NHCONH- or -NHCSNH-, respectively. Alternatively in Step A-4, treatment of **Va** with an alcohol and a tertiary amine affords compounds of Formula **Xa** wherein -X- is -O₂CNH-. Furthermore, **IVa** may be directly acylated by an active ester, a succinimidylcarbonate, an isocyanate, or an isothiocyanate, and the like, in Step A-5, to give compounds of Formula **Xa,** wherein -X- is -CONH-, -O₂CNH-, -NHCONH-, or -NHCSNH-. Strategies for the inclusion of additional protection and deprotection steps in order to control the desired regio-chemical outcome in the steps depicted for Path A are familiar to those having ordinary skill in the art.

When -X- is -NHCO₂- or -OCO₂-, the compounds of Formula **Xa** may be prepared from BCN-methanol **(*vi(a)*)** in two steps via Path B. In Step B-1, ***vi(a)*** is treated with N,N'-disuccinimidyl carbonate, to afford **VIIa** according to the previously disclosed method of Dommerholt et al., Angewande Chemie, International Edition, 2010, 49, 9422-9425. Subsequent treatment with a primary amine or an alcohol and a tertiary amine such as N-methylmorpholine, triethylamine, or diisopropylethylamine in Step B-2 affords a compound of Formula **Xa.** Strategies for the inclusion of additional protection and deprotection steps in order to control the desired regio-chemical outcome in the steps depicted for Path B are familiar to those having ordinary skill in the art.

When -X- is -O-, -NH-, or -S-, the compounds of Formula **Xa** may be prepared from BCN-methanol **(*vi(a)*)** in two steps via Path C. In Step C-1, the hydroxyl group of ***vi(a)*** is converted to a leaving group, ¹X-, such as a tosylate, a mesylate, an iodide, a bromide, or a chloride, to provide **Villa..** For example, treatment with a sulfonyl chloride or a sulfonic anhydride and an organic base such as pyridine, collidine, N-methylmorpholine, triethylamine, or diisopropylethylamine provides **Villa** wherein ¹X- is tosylate or mesylate. Alternatively, treatment with triphenylphosphine and a halogen reagent such as I₂, N-bromosuccinimide, carbon tetrabromide, N-chlorosuccinimide, or carbon tetrachloride provides **Villa,** wherein ¹X- is I, Br, or Cl. Displacement of the leaving group in Step C-2 by an O, N, or S nucleophile provides a compound of Formula **Xa.** Strategies for the inclusion of additional protection and deprotection steps in order to control the desired regio-chemical outcome in the steps depicted for Path C are familiar to those having ordinary skill in the art.

When -X- is -CO₂-, -NHCO₂- or -OCO₂-, the compounds of Formula **Xa** may be prepared from BCN-methanol **(*vi(a)*)** in two steps via Path D. In Step D-1, the hydroxyl group of ***vi(a)*** is acylated by a acylating reagent, such as a carboxylic acid chloride, a carboxylic acid anhydride, an active ester, an isocyanate, or a chloroformate, bearing a pendant silyl-protected hydroxyl group, in combination with an organic base such as pyridine, collidine, 4-dimethylaminopyridine, N-methylmorpholine, triethylamine, or diisopropylamine to provide **IXa.** Removal of the silyl protecting group by treatment with tetrabutylammonium floride, potassium fluoride, triethylammonium hydrofluoride, or pyridinium hydrofluoride affords a compound of Formula **Xa.** Strategies for the use of alternative protecting groups and deprotection conditions in Path D are familiar to those having ordinary skill in the art.

In the Final Step, the penultimate intermediate, **Xa,** which may be prepared by Path A, B, C or D, is converted to a compound of Formula **I** by treatment with a reactive phosphorous(III) reagent. For example, treatment of **Xa** with (*i*-Pr₂N)₂POCH₂CH₂CN, also known as "bis-reagent", and an acid catalyst, such as tetrazole, provides a compound of Formula **I** in which R¹- is N≡CCH₂CH₂O- and R²- is (*i*-Pr)₂N. Similarly for example, treatment of **Xa** with *i*-Pr₂NP(Cl)OCH₂CH₂CN, also known as "chloro-reagent", and a tertiary amine, such as diisopropylethylamine, provides also a compound of Formula **I** in which R¹- is N≡CCH₂CH₂O- and R²- is (*i*-Pr)₂N.

It is recognized by those with ordinary skill in the art of organic synthesis that BCN-ethanol **(*vi(c)*)** or BCN-propanol **(*vi(d)*)** may be substituted for BCN-methanol **(*vi(a)*)** as the starting material in Scheme 4a, thereby affording homologous intermediates of Formulae **Xc** and **Xd,** respectively, and homologous compounds of Formula **I** as the final product.

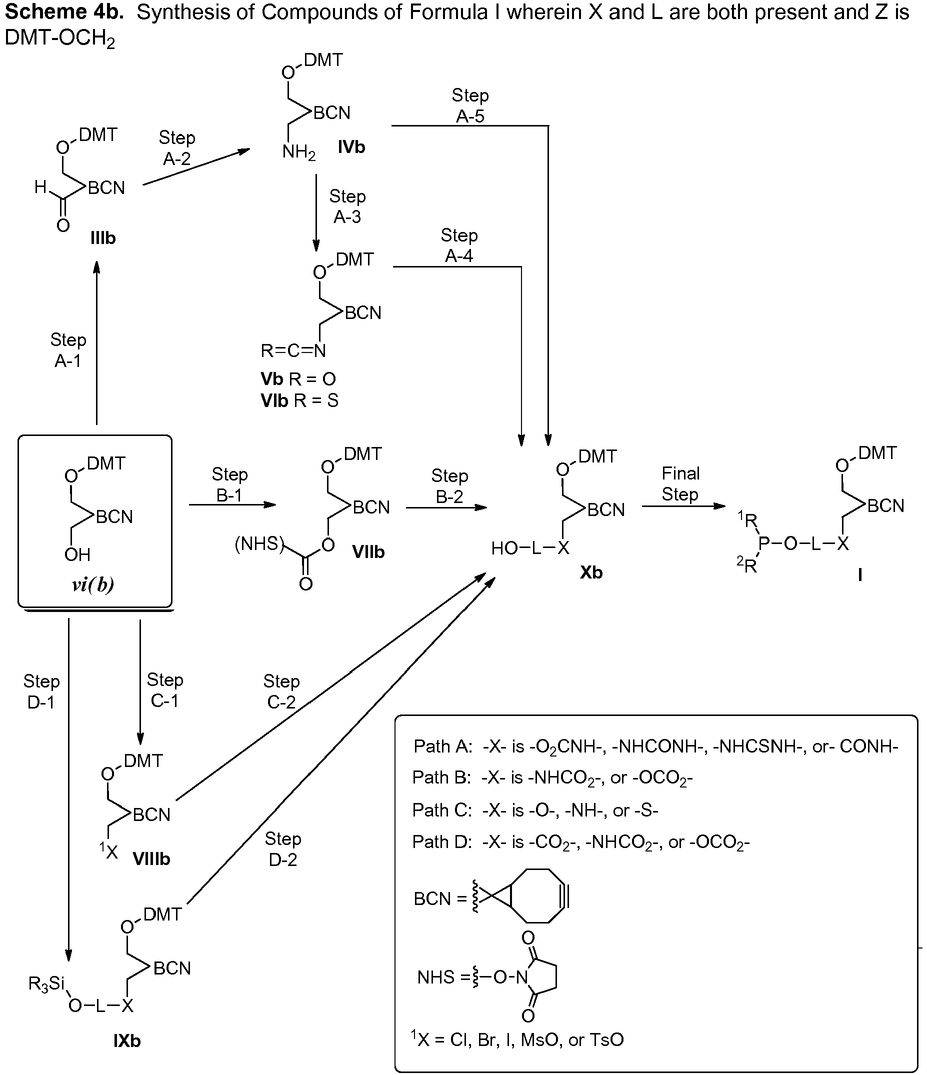

Scheme 4b illustrates the synthesis of the compounds of Formula I when X and L are both present, and Z is DMT-OCH₂. The synthetic transformations of Paths A, B, C, D as described above for Scheme 4a are analogously applicable to Scheme 4b. Thus, Paths A, B, C, and D convert DMT-OCH₂-BCN-methanol **(*vi(b)*)** to a compound of Formula **Xb.** Subsequently, **Xb** is transformed into a compound of Formula **I** in the Final Step. It is recognized by those with ordinary skill in the art of organic synthesis that DMT-OCH₂-BCN-ethanol **(*vi(e)*)** or DMT-OCH₂-BCN-propanol **(*vi(f)*)** may be substituted for DMT-OCH₂-BCN-methanol **(*vi(b)i)*** as the starting material in Scheme 4b, thereby affording homologous intermediates of Formulae **Xe** and **Xf,** respectively, and homologous compounds of Formula I as the final product.

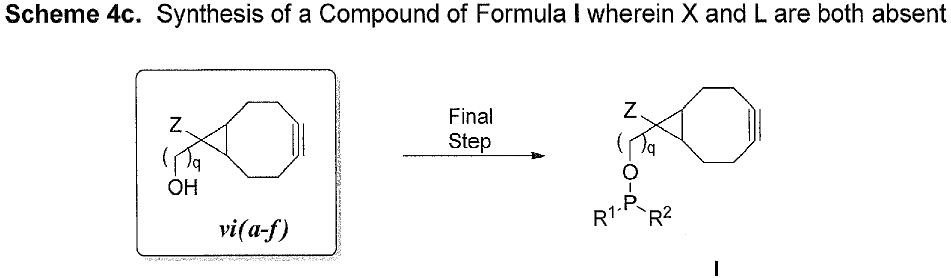

Scheme 4c illustrates the synthesis of a compound of Formula **I** from ***vi**(**a***-***f**)* when X and L are both absent. The starting alcohol, BCN-methanol **(*vi(a)*)*,*** DMT-OCH₂-BCN-methanol (***vi(b)***)***,*** BCN-ethanol (***vi(c)***)*,* DMT-OCH₂-BCN-ethanol (***vi(e)***)*,* BCN-propanol (***vi(d)***)*,* or DMT-OCH₂-BCN-propanol *(**vi(f)**),* is subjected to the conditions of the Final Step as described above for Schemes 4a and 4b.

### METHODS OF INCORPORATING BCN-CONTAINING COMPOUNDS OF FORMULA I INTO OLIGONUCLEOTIDES

The BCN-containing compounds of Formula **I** may be employed in automated DNA synthesis to introduce the BCN group into DNA oligonucleotides. As will be appreciated by those skilled in the art of DNA synthesis, such automated synthesis may be conducted as follows: (1) a desired DNA sequence is programmed into an automated DNA synthesizer that has been equipped with the necessary reagents; (2) the synthesizer carries out the synthesis steps in automated fashion over a number of cycles, adding each prescribed nucleotide of the sequence until the full-length, protected oligonucleotide is prepared on a solid support; and (3) the oligonucleotide is cleaved from the solid support, and protecting groups are removed, to give the free oligonucleotide.

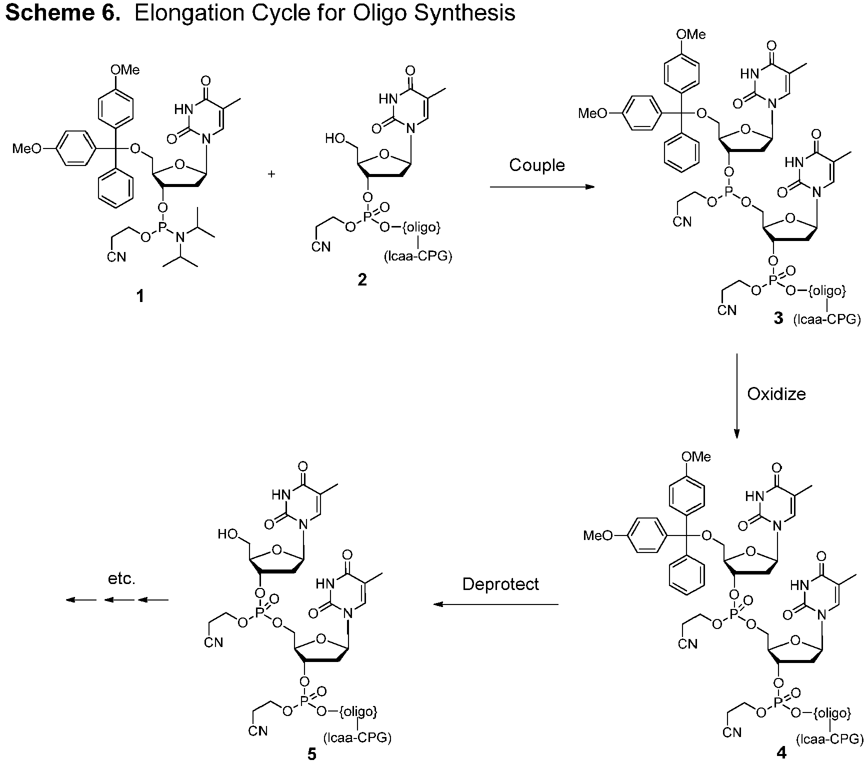

As shown in Scheme 6, the first nucleoside is attached to the solid support (e.g., CPG) via a cleavable linkage (e.g. Icaa or aminopropyl). The oligo is subsequently elongated using successive cycles of coupling, oxidation, and deprotection reactions. In the coupling reaction, the incoming nucleoside is in the form of its 5'-dimethoxytrityl-protected-3'-phosphoramidite, **1.** The phosphoramidite provides a reactive P(III) group which efficiently couples with the terminal hydroxyl group on **2** via displacement of diisopropyl amine. The resulting phosphite linkage of **3** is then oxidized to a phosphate linkage of **4.** Removal of the dimethoxytrityl ("DMT") protecting group readies the oligo, **5,** for the next incoming phosphoramidite. The full length oligo is cleaved from the support and all protecting groups are removed, affording an oligo of reasonable purity for many purposes.

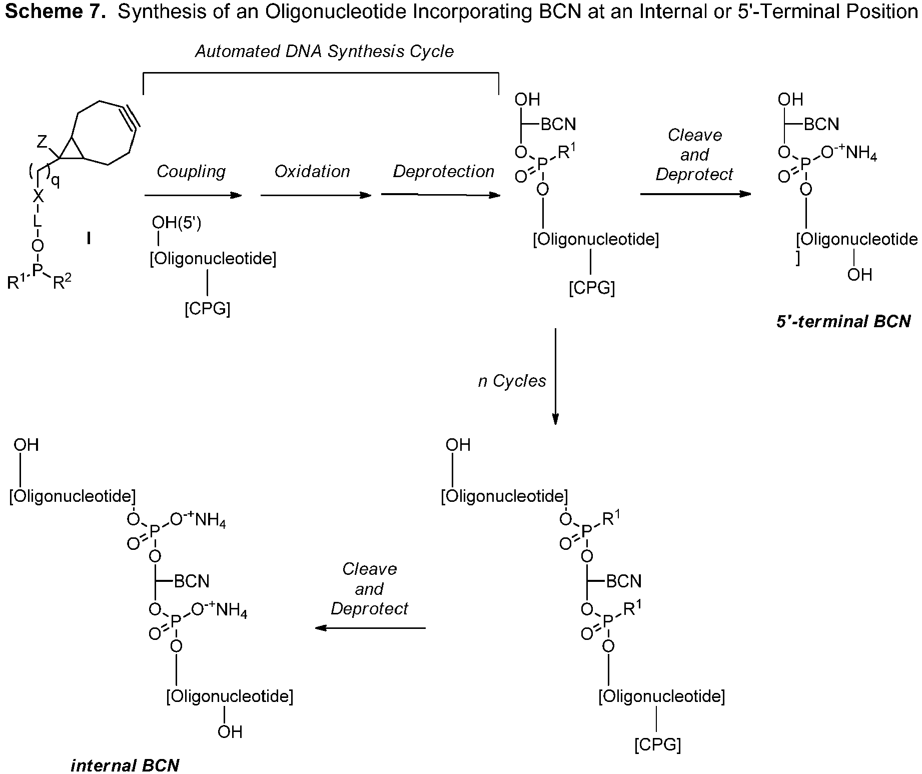

Scheme 7 illustrates a method for the introduction of a BCN-containing compound of Formula I at an internal or 5'-terminal position of an oligonucleotide. To obtain an oligonucleotide containing the BCN group at an internal position, DNA synthesis is continued after the incorporation of the compound of Formula I. To obtain an oligonucleotide containing the BCN group at the 5'-terminal position, the oligonucleotide is cleaved from the solid support after the incorporation of the compound of Formula I, and the protecting groups are removed, to afford the free oligonucleotide.

### LIGATION OF CHEMICAL TAGS TO BCN-CONTAINING OLIGONUCLEOTIDES

Scheme 9 depicts proof of concept studies for the conjugation of various cofactor-azides with a 5'-BCN-containing oligonucleotide incorporating a compound of Formula I. An oligo consisting of six thymidine nucleotides is terminated with **10** using the elongation cycle of chemical reactions described above (Scheme 4) to afford **5'-(10)-(T₆)-Icaa-CPG.** This is achieved using the solid supported synthesis method in an automated oligonucleotide synthesizer. The oligo is cleaved from the solid support and cyanoethyl protecting groups are removed from the phosphate groups to afford **5'-(10)-(T₆).** These operations are achieved through the use of methods that are well known to those skilled in the art of oligonucleotide synthesis.

The resulting 5'-BCN-containing oligonucleotide **(5'-(10)-(T₆))** is dissolved in a mixture of an aqueous buffer and a water-miscible organic solvent such as acetonitrile. A solution of the cofactor-azide is then added, and the mixture is allowed to stand at room temperature. The Cu-free click reaction proceeds cleanly, affording the cofactor-oligo conjugate. The progress of the conjugation reaction can be followed by HPLC and the identity of the conjugate can be confirmed by mass spectrometry. The flexibility and robustness of this approach is shown by the preparation of **37, 38,** and **39,** in which diverse chemical tags have been incorporated using the catalyst-free click reaction.

Other compounds of Formula I, such as **11, 22, 23, 24, 25, 48, 49, 50, 51,** and **54,** may be employed under analogous conditions to prepare other 5'-BCN-containing oligonucleotides. Yet other compounds of Formula I, such as **7a, 7b, 13, 15, 19, 21, 42, 43,** and **47,** may also be employed to prepare BCN-containing oligonucleotides bearing BCN groups at internal positions (e.g. (T₅)-(**I**)-(T₅) or A-T-G-C-C-G-T-A-(**I**)-T-A-G-C). Regardless of the structure or position of the BCN group in the BCN-containing oligonucleotide, the Cu-free click reactions may be carried out as described above to attach a variety of chemical tags to the oligonucleotides. Further details pertaining to the use of compounds of the invention in oligonucleotide tagging are provided in the Examples.

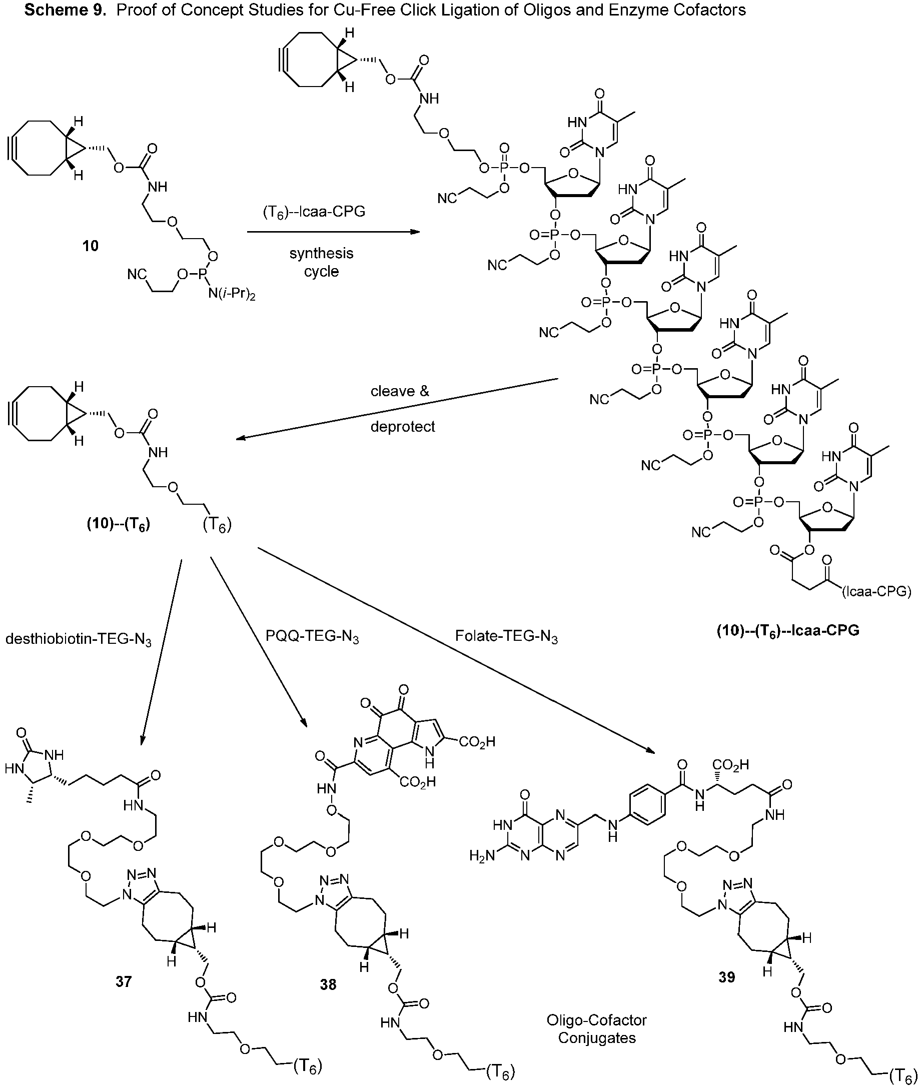

### EXAMPLES

### Example 1: endo-Bicyclo[6.1.0]non-4-yn-9-ylmethyl (2-(2-(((2-cyanoethoxy)(diisopropylamino)-phosphino)oxy)ethoxy)ethyl)carbamate, 10.

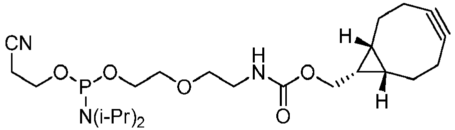

A solution of *endo*-bicyclo[6.1.0]non-4-yn-9-ylmethyl (2-(2-hydroxyethoxy)ethyl)carbamate(hereinafter **9**) (0.42 g, 1.5 mmol) (obtained from SynAffix, B.V, Catalog No. SX-A1012) in anhydrous dichloromethane (7 mL) is treated with 3-((bis(diisopropylamino)phosphino)-oxy)propanenitrile (0.55 mL, 1.7 mmol). The resulting solution is treated with a dichloromethane solution containing 0.25M trifuoroacetic acid and 0.5M N-methylmorpholine (3.0 mL, 0.75 mmole H⁺). The reaction is stirred at room temperature for 90 minutes. After dilution with dichloromethane (20 mL), the reaction solution is washed with water (2 X 25 mL) and then washed with 5% aqueous NaHCO₃ (1 X 25 mL). The organic layer is dried over Na₂SO₄, filtered, and evaporated at reduced pressure. A slurry of Silica gel (5 g) in hexane-acetone-triethylamine (85:10:5) is packed into a 1.5 cm diameter column. The crude reaction product is dissolved in hexane-acetone-dichloromethane (80:10:10) and loaded onto the silica column. Elution with hexane-acetone-dichloromethane (80:10:10 followed by 70:20:10) and collection of 5 mL fractions allows the separation of **10** from other impurities. Pure fractions are evaporated to afford a colorless liquid (0.6g). MS (AP+): 482 (M+H); 504 (M+Na) is consistent with the desired phosphoramidite, **10.**

### Example 2: 2-(exo-Bicyclo[6.1.0]non-4-yn-9-yl)ethyl (2-cyanoethyl) diisopropylphosphoramidite, 11.

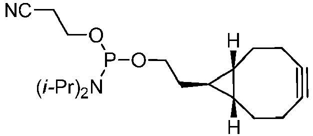

A solution of *exo*-BCN-ethanol (***vi(c)** from* Example 23, 2.0 g, 12.2 mmol) is treated with 3-((bis(diisopropylamino)phosphino)-oxy)propanenitrile (4.53 mL, 14.0 mmol) according to the method of Example 1 to afford **11,** as a colorless liquid. MS(AP+): 365 (M+H); 387 (M+Na) is consistent with the desired phosphoramidite, **11.**

### Example 3: endo-Bicyclo[6.1.0]non-4-yn-9-ylmethyl ((E)-3-(5'-O-dimethoxytrityl-2'-deoxyuridin-5-yl)allyl)carbamate, 12.

A solution of 5-((E)-3-aminoprop-1-en-1-yl)-5'-O-dimethoxytrityl-2'-deoxyuridine (1.17 g, 2.0 mmol) (prepared according to the method of Santoro, *et al., J. Am. Chem. Soc.* 2000, *122*(11), 2433-9)in DCM (10 mL) is treated with a solution of *endo-*bicyclo[6.1.0]non-4-yn-9-ylmethyl (2,5-dioxopyrrolidin-1-yl) carbonate (**VIIa**) (0.64 g, 2.2 mmol) (obtained from SynAffix, Catalog No. SX-A1028) in dichloromethane (10 mL). After stirring overnight at room temperature, the reaction mixture is washed with saturated aqueous NaHCO₃ and the organic layer is dried over Na₂SO₄. After filtration, the resulting solution is concentrated at reduced pressure. The residue is purified by chromatography on silica gel, eluting with a gradient of 1-5% methanol in dichloromethane. Fractions containing pure **12** are combined and concentrated at reduced pressure. The residue is dissolved in dichloromethane (50 mL) and concentrated again. Drying overnight under vacuum affords **12** as a crisp, colorless foam. MS (AP+): 762 (M+H).

### Example 4: endo-Bicyclo[6.1.0]non-4-yn-9-ylmethyl((E)-3-(3'-O-(((2-cyanoethoxy)-diisopropylamino)phosphityl)-5'-O-dimethoxytrityl-2'-deoxyuridin-5-yl)allyl)carbamate, 13.

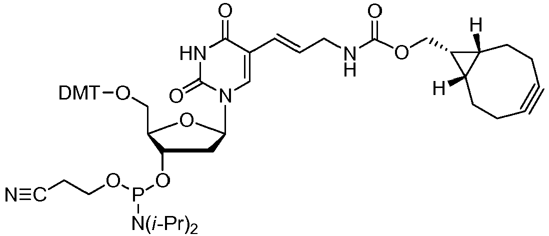

**12** (from Example 3) (1.14 g, 1.5 mmol) is dissolved in anhydrous dichloromethane (10 mL). The resulting solution is treated with with 3-((bis(diisopropylamino)phosphino)-oxy)propanenitrile (0.55 mL, 1.72 mmol), followed by addition of with a dichloromethane solution containing 0.25M trifluoroacetic acid and 0.5M N-methylmorpholine ( 3.0 mL, 0.75 mmole H⁺). The reaction solution is stirred for 4 hours at room temperature. After dilution with dichloromethane (25 mL), the reaction solution is washed with water (2 X 25 mL) and then washed with 5% aqueous NaHCO₃ (1 X 25 mL). The organic layer is dried over Na₂SO₄, filtered, and evaporated at reduced pressure. The residue is dissolved in dichloromethane (3 mL), diluted with n-pentane (3 mL) and the resulting solution is added dropwise to vigorously stirred n-pentane. The resulting suspension is allowed to stand for 15 minutes then the hazy liquor is decanted from the gummy precipitate that adheres to the flask walls. The gummy precipitate is dissolved in acetonitrile (20 mL). The solution is dried over Na₂SO₄, filtered, and concentrated at reduced pressure. The residue is dissolved in anhydrous dichloromethane (30 mL) and concentrated at reduced pressure. Further drying under vacuum overnight gives a **13** as a crisp, colorless foam. MS (AP+): 962 (M+H).

### Example 5: endo-Bicyclo[6.1.0]non-4-yn-9-ylmethyl(6-((E)-3-(3'-O-(((2-cyanoethoxy)-diisopropylamino)phosphityl)-5'-O-dimethoxytrityl-2'-deoxyuridin-5-yl)acrylamido)hexyl)carbamate, 15

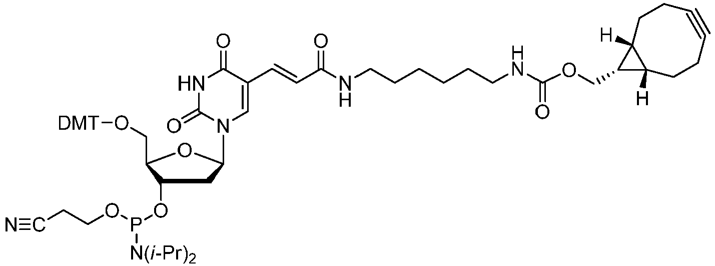

A solution of 5-[*N*-(6-Aminohexyl)-3-(*E*)-acrylamido]-5'-O-(dimethoxytrityl)-2'-deoxyuridine (obtained from Berry and Associates, Inc., Catalog No. PY 7050) is treated with **VIIa** according to the method of Example 3 to provide endo-Bicyclo[6.1.0]non-4-yn-9-ylmethyl(6-((E)-3-(5'-O-dimethoxytrityl-2'-deoxyuridin-5-yl)acrylamido)hexyl)carbamate (hereinafter **14**), which is then phosphitylated according to the method of Example 4 to afford **15.** MS (AP+): 1075, (M+H).

### Example 6: (RS)-endo-Bicyclo[6.1.0]non-4-yn-9-ylmethyl (8-dimethoxytrityloxy-6-hydroxy-4,8-dioxa-octyl)carbamate, 6a.

(R,S)-3-(3-aminopropoxy)propane-1,2-diol (1.64 g, 11 mmol) (Prepared according to the method of Misiura and Gait, WO 9117169 A1) and **VIIa** (2.91 g, 10 mmol) are dissolved in anhydrous tetrahydrofuran (50 mL) and stirred overnight at room temperature. The reaction solution is concentrated at reduced pressure and the residue is partitioned between ethyl acetate (50 mL) and 5% aqueous Na₂CO₃ (20 mL). The organic layer is dried over Na₂SO₄, filtered, and concentrated at reduced pressure. The residue is dissolved in anhydrous pyridine (30 mL) and treated with dimethoxytrityl chloride (3.0 g, 9.0 mmol) and the resulting solution is stirred overnight at room temperature. The reaction is concentrated at reduced pressure and the resulting residue is partitioned between ethyl acetate and saturated aqueous NaHCO₃. The organic layer is washed with saturated aqueous NaCl, dried over Na₂SO₄, filtered, and concentrated at reduced pressure. Flash chromatography on silica gel, eluting with a gradient of 5% to 45% ethyl acetate in hexanes affords **6a** as a sticky foam upon evaporation of solvents at reduced pressure. MS (AP+): 628, (M+H).

### Example 7: (RS)-endo-Bicyclo[6.1.0]non-4-yn-9-ylmethyl (8-dimethoxytrityl-6-(((2-cyanoethoxy)-diisopropylamino)phosphino)-4,8-dioxa-octyl)carbamate, 7a.

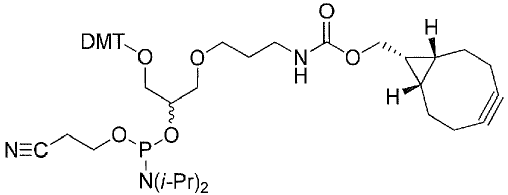

**6a** from Example 6 is phosphitylated according to the method of Example 1 to provide the phosphoramidite, **7a.** MS (AP+): 828 (M+H).

### Example 10: (1R,8S)-Diethyl bicyclo[6.1.0]non-4-ene-9,9-dicarboxylate (ii(b)).

To a solution of 1,5-cyclooctadiene (5.27 mL, 43.0 mmol) and Rh₂(OAc)₄ (100 mg, 0.23 mmol) in CH₂Cl₂ (5 mL) is added dropwise over 3 h a solution of diethyl diazomalonate (1.0 g, 5.37 mmol) in CH₂Cl₂ (5 mL). This solution is stirred for 24 h at room temperature. The CH₂Cl₂ is evaporated and the excess of cyclooctadiene is removed by filtration over a glass filter filled with silica (eluent: heptane). The filtrate is concentrated *in vacuo* and the residue is purified by column chromatography on silica gel (ethyl acetate:heptane, 1:10) to afford ***ii(b)*** (1.03 g, 72%). ¹H NMR (CDCl₃, 400 MHz): δ□15.65-5.57 (m, 2H), 4.10 (2×q, *J* = 7.2 Hz, 4H), 2.41-2.29 (m, 2H), 2.15-2.06 (m, 3H), 1.83-1.70 (m, 3H), 1.31-1.23 (2xt, *J* = 7.2 Hz, 6H).

### Example 11: (1R,8S)-Bicyclo[6.1.0]non-4-ene-9,9-diyldimethanol (iii(b))

To a suspension of LiAlH₄ (103 mg, 2.70 mmol) in diethyl ether (10 mL) is added dropwise at 0 °C a solution of ***ii(b)*** from Example 10 (400 mg, 1.50 mmol) in diethyl ether (10 mL). Water is added carefully until the grey solid turns white. Na₂SO₄ (2 g) is added and the solid is filtered-off and washed thoroughly with diethyl ether (100 mL). The filtrate is concentrated *in vacuo.* The residue is purified by column chromatography on silica gel (ethyl acetate:heptane, 3:1) to afford ***iii(b)*** as a white solid (190 mg, 69%). ¹H NMR (CDCl₃, 400 MHz): δ 5.66-5.58 (m, 2H), 3.88 (d, *J* = 4.8 Hz, 2H), 3.58 (d, *J* = 4.8 Hz, 2H), 2.43-2.35 (m, 2H), 2.20-1.99 (m, 6H), 1.71-1.57 (m, 2H), 0.95-0.88 (m, 2H).

### Example 12: ((1R,8S)-4,5-Dibromobicyclo[6.1.0]nonane-9,9-diyl)dimethanol (iv(b))

The diol, ***iii(b)*** from Example 11, (145 mg, 0.796 mmol) is dissolved in CH₂Cl₂ (5 mL). At 0 °C a solution of Br₂ (45 µL, 0.875 mmol) in CH₂Cl₂ (1 mL) is added dropwise until the yellow color persists. The reaction mixture is quenched with a 10% Na₂S₂O₃ solution (5 mL) and extracted with CH₂Cl₂ (2 x 20 mL). The organic layer is dried (Na₂SO₄) and concentrated *in vacuo.* The residue is purified by column chromatography on silica gel (EtOAc:heptane, 5:1) afford ***iv(b)*** (235 mg, 86%) as a white solid. ¹H NMR (CDCl₃, 400 MHz): δ 4.87-4.78 (m, 2H), 3.96-3.88 (m, 2H), 3.60 (d, *J* = 5.2 Hz, 2H), 2.75-2.63 (m, 2H), 2.32-2.22 (m, 3H), 2.20-2.13 (m, 1H), 2.05-1.94 (m, 2H), 1.74-1.57 (m, 2H), 1.13-0.99 (m, 2H).

**Example 13:** BCN-dimethanol, ***v(b)**.* To a solution of the dibromide, ***iv(b)*** from Example 12, (100 mg, 0.292 mmol) in THF (5 mL) is added dropwise at 0 °C a solution of KO*t*Bu (1.29 mL, 1 M in THF, 1.29 mmol). The solution is then refluxed for 1.5 h. After cooling to room temperature, the mixture is quenched with saturated NH₄Cl-solution (20 mL), and extracted with CH₂Cl₂ (3 x 20 mL). The organic layer is dried (Na₂SO₄) and concentrated *in vacuo.* The residue is purified by column chromatography on silica gel (ethyl acetate) to afford ***v(b)*** (24 mg, 46%) as a white solid. ¹H NMR (CDCl₃, 400 MHz): δ 3.89 (bs, 2H), 3.63 (bs, 2H), 2.58 (bs, 2H), 2.34-2.20 (m, 6H), 1.68-1.59 (m, 2H), 0.89-0.82 (m, 2H).

### Example 14: (9-((dimethoxytrityloxy)methyl)bicyclo[6.1.0]non-4-yn-9-yl)methanol (vi(b))

A solution of ***v(b)*** from Example 13 (1.8 g, 10 mmol) is dissolved in anhydrous pyridine (50 mL) and treated with small portions of DMT-Cl (10 X 339 mg, 10 mmol) at 20 minute intervals. The resulting solution is stirred for an additional 3 hours. After concentration at reduced pressure, the residue is partitioned between ethyl acetate (100 mL) and saturated aqueous NaHCO₃ (50 mL). The organic layer is washed with saturated aqueous NaCl (50 mL), dried over Na₂SO₄, filtered, and concentrated at reduced pressure. The residue is purified by chromatography on silica gel, eluting with a gradient of 10-60% ethylacetate in hexanes to afford ***vi(b)*** upon evaporation of solvents. MS (AP+): 483 (M+H).

### Example 15: 2-(endo-bicyclo[6.1.0]non-4-yn-9-yl)ethyl (3-(3-(bis(4-methoxyphenyl)(phenyl)methoxy)-2-(((2-cyanoethoxy)(diisopropylamino)phosphino)oxy)propoxy)propyl)carbamate (42).

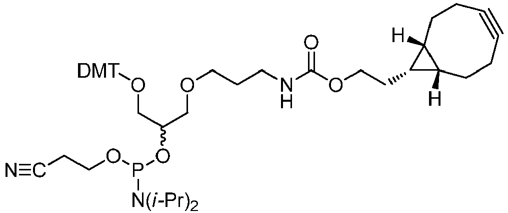

(RS) 2-(*endo*-Bicyclo[6.1.0]non-4-yn-9-yl)ethyl (3-(3-(bis(4-methoxyphenyl)(phenyl)methoxy)-2-hydroxypropoxy)propyl)carbamate **(41)** is first prepared by substituting **40e,** from Example 25 for **Vila** in the method of Example 6. Purified **41** is then phosphitylated according to the method of Example 1 to provide the phosphoramidite, **42.** MS (AP+): 843 (M+H).

### Example 16: endo-Bicyclo[6.1.0]non-4-yn-9-yl-formaldehyde (17)

To a cooled (0 °C) suspension of BCN-methanol (180 mg, 1.20 mmol) in CH₂Cl₂ (10 mL) is added Dess-Martin periodinane (0.68 g, 1.6 mmol) and the suspension is stirred for 4 h at rt. After this time, water (10 mL) is added, the solvent layers are separated and the CH₂Cl₂ layer is dried on MgSO₄ and filtered. The filtrate is concentrated *in vacuo* at 0 °C and the residue is purified by column chromatography on silica gel (CH₂Cl₂) to afford **17** as a white solid (53 mg, 30%). ¹H NMR (CDCl₃, 300 MHz): δ9.6 (d, 1 H), 2.33-2.22 (m, 9H), 1.57-1.54 (m, 3H).

### Example 17: endo-Bicyclo[6.1.0]non-4-yn-9-yl-methylamine (18)

To a stirred solution of *endo*-bicyclo[6.1.0]non-4-yn-9-ylformaldehyde (15 mg, 0.10 mmol) in MeOH (5 mL) is added NH₄OAc (0.50 g) and NaCNBH₃ (7.5 mg, 0.12 mmol). After stirring overnight, the mixture H₂O (10 mL) is added and the resulting solution is concentrated *in vacuo.* Diethyl ether (10 mL) and saturated Na₂CO₃ (1 mL) are added, the mixture shaken and the layers are allowed to separate. The aqueous phase is extracted with CH₂Cl₂ (2x 10 mL). The combined organic layers are dried (MgSO₄) and filtered. The filtrate is concentrated *in vacuo,* applied onto a column of silica gel and eluted with a mixture of MeOH/NH₃ (7 N in MeOH)/CH₂Cl₂ (1:1:48→3:1:46→3:3:44). Pure fractions are concentrated, re-dissolved in CH₂Cl₂ and concentrated again to afford pure **18** (9 mg, 60%). ¹H NMR (CDCl₃): δ 2.75 (d, 1H), 2.26-2.16 (m, 3H), 1.65-1.50 (m, 1H), 1.40-1.08 (m, 6H), 0.90-0.82 (m, 2H).

### Example 18: (RS)-endo-bicyclo[6.1.0]non-4-yn-9-ylmethyl-(3-((3-(((2-cyanoethoxy)(diisopropylamino)phosphino)oxy)-5-dimethoxytrityloxypentyl)oxy)propyl)carbamate (7b).

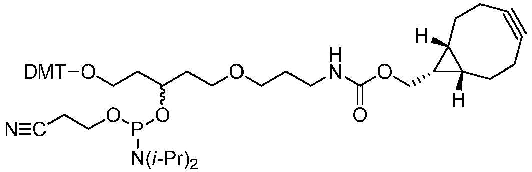

The reaction of (R,S)-5-(3-aminopropoxy)pentane-1,3-diol (synthesized from 1,3,5-pentanetriol (Obtained from Beta Pharma Scientific Products, Catalog No. 86-43517) in 4 steps according to the analogous method of Misiura and Gait, WO 9117169 A1) under the conditions of Example 6 affords (RS)-endo-bicyclo[6.1.0]non-4-yn-9-ylmethyl-(3-((3-hydroxy-5-dimethoxytrityloxypentyl)oxy)propyl)carbamate (hereinafter **6b**), which is phosphitylated according to the method of Example 7 to afford **7b.**

### Example 21: (Z)-exo-Bicyclo[6.1.0]non-4-ene-9-carbaldehyde, exo-iii(c).

(Z)-*exo*-Bicyclo[6.1.0]non-4-en-9-ylmethanol (*exo-**iii(a)***) (prepared according to Dommerholt et al., Angewandte Chemie, International Edition, 2010, 49, 9422-9425) (5.2 g, 26.6 mmol) is dissolved in dichloromethane (300 mL). Pyridinium chlorochromate (10.5 g, 48.5 mmol) is added. The resulting reaction mixture is stirred for 2 hours and subsequently filtered over a short path of silicagel. The filtrate is concentrated and purified by column chromatography (dichloromethane), yielding 4.9 g of the aldehyde, *exo-**iii(c)**.* This material is used without further purification in Example 22.

### Example 22: (Z)-exo-2-(Bicyclo[6.1.0]non-4-en-9-yl)ethanol, exo-iv(c).

Under an atmosphere of argon, (methoxymethyl)triphenylphosphonium chloride (17.1 g; 50 mmol) is suspended in anhydrous THF (100 mL) and cooled to 0°C. Potassium *tert-*butoxide (5.6 g; 50 mmol) is added and the resulting mixture is stirred for 20 minutes. A solution of *exo-**iii(c)*** (Example 21, 4.95 g; 33.0 mmol) in anhydrous THF (100 mL) is added. The resulting reaction mixture is stirred for 15 minutes and then poured into a mixture of diethylether and water (200 mL/200 mL). The aqueous phase is separated and extracted a second time with diethylether (100 mL). The two combined organic layers are dried (Na₂SO₄) and concentrated at reduced pressure. The residue is dissolved in tetrahydrofuran (200 mL) and aqueous hydrochloric acid (1M, 100 mL) is added. The resulting mixture is heated to reflux for 45 minutes, cooled to room temperature and poured into a mixture of diethylether and water (200 mL/200 mL). The aqueous phase is separated and extracted a second time with diethylether (100 mL). The two combined organic layers are dried (Na₂SO₄) and concentrated at reduced pressure. The residue is dissolved in methanol (200 mL) and placed under an atmosphere of argon. After cooling the reaction mixture to 0°C, NaBH₄ (1.89 g; 50 mmol) is added in portions. The mixture is stirred for 15 minutes, quenched with saturated aqueous ammonium chloride (100 mL) and partitioned between diethylether (200 mL) and water (100 mL). The aqueous phase is separated and extracted with diethylether (2 × 200 mL). The three combined organic layers are dried (Na2SO4) and concentrated at reduced pressure. The crude product is purified by column chromatography on silica gel, eluting with a 10-25% gradient of ethylacetate in pentane to provide 4.22 g (77%) of *exo-**iv(c)**.* ¹H-NMR (300 MHz, CDCl₃) δ 5.70 - 5.56 (m, 2H), 3.68 (t, *J* = 6.6 Hz, 2H), 2.39 - 1.94 (m, 6H), 1.51 (q, *J* = 6.7 Hz, 2H), 1.44 - 1.23 (m,3H), 0.71 - 0.57 (m, 2H), 0.30-0.20 (m, 2H).

### Example 23: exo-BCN-ethanol, exo-vi(c).

A solution of bromine (1.37 mL, 26.7 mmol) in dichloromethane (25 mL) is added dropwise to an ice-cold solution of *exo-**iv(c)*** (Example 22, 4.22 g, 25.4 mmol) in dichloromethane (100 mL). Subsequently, 10% aqueous Na₂S₂O₃ (50 mL) is added. The aqueous phase is separated and extracted a second time with dichloromethane (50 mL). The two combined organic layers are then dried (Na₂SO₄) and concentrated at reduce pressure to afford *exo-**v(c)**,* 8.33 g (100%). Without further purification, *exo-**v(c)*** is dissolved in anhydrous THF (100 mL), placed under an argon atmosphere, and cooled to 0°C. A solution of potassium *tert*-butoxide (9.3 g; 83 mmol) in anhydrous THF (100 mL) is added dropwise. The resulting reaction mixture is heated to 70°C, stirred for 30 minutes, and quenched with saturated NH₄Cl_{(aq)}(100 mL). The resulting mixture is extracted twice with diethylether (200 mL). The two combined organic layers are then dried (Na₂SO₄) and concentrated at reduced pressure. The crude product is purified chromatography on silica gel to afford exo-***vi(c)***, (2.57 g; 15.6 mmol; 62%) as a slightly yellow solid/wax. ¹H NMR (300 MHz, CDCl₃) δ 3.71 (t, *J* = 6.5 Hz, 2H), 2.46 - 2.07 (m, 6H), 1.63-1.54 (m,2H), 1.44 - 1.22. *endo-**vi(c)*** is likewise produced from produced from *endo-**iv(c)**. endo-**iv(c)**,* in turn, is obtained from *endo-**iii(a)*** (prepared according to Dommerholt et al., Angewandte Chemie, International Edition, 2010, 49, 9422-9425) according to the procedures of Examples 21 and 22.

### Example 24: exo-BCN-propanol, vi(d).

Starting with ***iv(c)*** from Example 22, the methods of Examples 21-23 are employed to provide ***vi(d).***

### Example 25 2-(endo-bicyclo[6.1.0]non-4-yn-9-yl)ethyl (2,5-dioxopyrrolidin-1-yl) carbonate(40e).

Under ambient atmosphere, *endo-**vi(c)*** (Example 23, 83 mg; 0.51 mmol) is dissolved in acetonitrile (10 mL). N,N'-disuccinimidylcarbonate (230 mg; 0.90 mmol) is added, followed by triethylamine (213µL; 155 mg; 1.53 mmol). The resulting mixture is stirred for 4 hours at room temperature. Ethylacetate (20 mL) is then added and the organic mixture is washed with water (3 × 20 mL), dried (Na₂SO₄) and concentrated. The crude is purified with column chromatography (ethylacetate/pentane 1/2), yielding 114 (0.37 mmol; 73%)mg of **40e.** ¹H NMR (300 MHz, CDCl₃) δ 4.37 (t, *J* = 6.8 Hz, 2H), 2.84 (s, 4H), 2.39 - 2.14 (m, 6H), 1.83-1.74 (m, 2H), 1.62-1.38 (m,3H), 1.07 - 0.93 (m, 1 H), 0.92-0.78 (m, 2H). 2-(*exo*-bicyclo[6.1.0]non-4-yn-9-yl)ethyl (2,5-dioxopyrrolidin-1-yl) carbonate(**40x**) is likewise produced from *exo**-vi(c)**.*

### Example 26 3-((((exo-bicyclo[6.1.0]non-4-yn-9-yl)ethoxy)propyl)disulfanyl)propyl (2-cyanoethyl) diisopropylphosphoramidite (49).

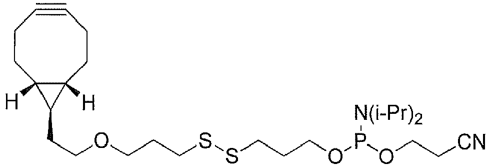

A solution of *exo-**vi(c)*** from Example 23 (1.64g, 10 mmol) in dichloromethane (50 mL) is treated with triethylamine (1.67 mL, 12 mmol) and methanesulfonyl chloride (0.85 mL, 11 mmol), stirring overnight at room temperature. The resulting solution is washed successively with water (50 mL), 0.1 M aqueous KH₂PO₄ (50 mL), and water (50 mL). The dichloromethane layer is dried over Na₂SO₄, and filtered. The resulting solution is concentrated at reduced pressure to provide 2-(exo-bicyclo[6.1.0]non-4-yn-9-yl)ethyl methanesulfonate (2.4 g). This material is dissolved in anhydrous THF (25 mL) and added to a solution formed by the mixing 3,3'-disulfanediylbis(propan-1-ol) (5.5 g, 30 mmol), potassium-*t*-butoxide (3.3 g, 29.5 mmol) and anhydrous THF (60 mL). The reaction is heated at reflux under a nitrogen atmosphere for 3 hours. After cooling to room temperature, water (2 mL) is added and the reaction mixture is concentrated to half its original volume at reduced pressure. The concentrate is partitioned between ethyl acetate (100 mL), hexane (100 mL) and water (100 mL). The organic layer is dried over Na₂SO₄ and filtered. The resulting solution is concentrated at reduced pressure. The crude product is purified by chromatography on silica to afford 3-((3-(2-(*exo-*bicyclo[6.1.0]non-4-yn-9-yl)ethoxy)propyl)disulfanyl)propan-1-ol (1.9 g). This material is treated according to the method of Example 1 to afford **49,** as a viscous oil. MS(AP+): 529 (M + H), 551 (M + Na).

### Example 27: 2-(endo-bicyclo[6.1.0]non-4-yn-9-yl)ethyl (2-(2-(((2-cyanoethoxy)(diisopropylamino)phosphino)oxy)ethoxy)ethyl)carbamate (54).

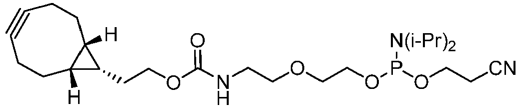

Under ambient atmosphere, **40e** (112 mg; 0;.37 mmol) is dissolved in dichloromethane (5 mL). Triethylamine (154 µL; 112 mg; 1.11 mmol) and 2-(2-aminoethoxy)ethanol (56 µL; 59 mg; 0.56 mmol) are added. The mixture is stirred for 30 minutes at room temperature and then diluted with Et₂O (30 mL). The organic mixture is washed with a saturated aqueous solution of ammonium chloride (2 × 30 mL), washed with a saturated aqueous solution of sodium bicarbonate (2 × 30 mL), dried and concentrated, yielding 82 mg (0.28 mmol; 76%) mg of 2-(endo-bicyclo[6.1.0]non-4-yn-9-yl)ethyl (2-(2-hydroxyethoxy)ethyl)carbamate, **53** as a colorless syrup. Subsequently, **53** is treated according to the procedure of Example 1 to provide **54.** MS (AP+): 496 (M+H); 5518 (M+Na).

### Example 28: 5'-BCN-T₆-oligo

Using a Millipore Expedite (8900 series) nucleic acid synthesis system (Billerica, MA), freshly prepared reagent solutions are installed in the reagent bottles as follows:
- Wash A - anhydrous acetonitrile
- Deblock - 3% Trichloroacetic acid in anhydrous dichloromethane
- Oxidizer - 0.02M iodine in tetrahydrofuran/water/pyridine
- Capping reagent A - acetic anhydride/anhydrous tetrahydrofuran
- Capping reagent B - 16% 1-methylimidazole in anhydrous tetrahydrofuran/pyridine
- Wash reagent - anhydrous acetonitrile
- Activator - 0.25M 5-ethylthiotetrazole in anhydrous acetonitrile
- Amidites: Thymidine-CEP and compound **10** from Example 1 (0.067M solutions in anhydrous acetonitrile)

The reagent lines were purged and pumps primed. Two synthesis columns containing 200 nM of DMT-T-Icaa-CPG were installed.

The instrument run parameters were then set as follows:
Column - 1:
   - Sequence - 3'-TTTTTTX-5' (wherein T denotes a Thymidine residue and X denotes the BCN tag derived from **10**.)
   - Protocol - CYCLE T (a 23 step protocol for reagent additions, reaction times, and washes known to be optimized for each coupling of Thymidine-CEP, as provided in the synthesizer software.)
   - Final DMT - On (The BCN tag is not subjected to DMT cleavage reagent since DMT protection is not present.)
Column - 2:
   - Sequence - 3'-TTTTTT-5'
   - Protocol - CYCLE T
   - Final DMT - Off
5'-BCN-T₆-Icaa-CPG is synthesized in column 1 using CYCLE T conditions for each T residue and for the final coupling of **10.** T₆-Icaa-CPG is synthesized in column 2 using CYCLE T conditions for each T residue. The output of the colorimetric monitoring of each deblock step is recorded by the synthesizer's computer. The integrated values for each of the 6 deblock steps are consistent with the successful synthesis of T₆-Icaa-CPG on both columns. In order to verify that the coupling of **10** was successful, each column is washed twice with 3 mL of 10% diethylamine in acetonitrile at room temperature, washed with 3 mL of acetonitrile, and treated with 3 mL of 28-30% ammonium hydroxide for 15 minutes at room temperature in order to remove the cyanoethyl protecting groups and cleave the oligonucleotide from the CPG support. The resulting solutions of 5'-BCN-T₆-oligo and T₆-oligo are each treated with 25 uL of triethylamine and then sparged with a stream of nitrogen until the volume was reduced to approximately 1.5 mL. The concentrated solutions are then frozen and lyophilized. Reversed phase HPLC analysis on a Waters Spherisorb ODS-2 column (150 X 4.6 mm) eluting at 1.0 mL/min with a 30 minute gradient of 5 to 35% acetonitrile in 0.1 M triethylammonium acetate shows a retention time for T₆-oligo of 11.3 minutes (DNA product from column 2) and a retention time for 5'-BCN-T₆-oligo of 18.1 minutes (DNA product from column 1). Furthermore, an integration ratio of 99 (5'-BCN-T₆-oligo) to 1 (T₆-oligo) is observed for the peaks in the HPLC chromatogram of DNA product from column 1, thereby confirming the successful coupling of **10** at the 5'-terminus of the oligonucleotide with high efficiency.

### Example 29: Cu-free click conjugation of Desthiobiotin-TEG-Azide and 5'-BCN-T₆-oligo to provide 37.

A solution composed of 5'-BCN-T₆-oligo (-90 nmol), desthiobiotin-TEG-azide (Berry & Associates, Inc. catalog no. BT 1075, 2400 nMol), 0.1 M aqueous triethylammonium acetate (pH 7, 0.75 mL) and acetonitrile (0.15 mL) is allowed to stand at room temperature. The progress of the Cu-free click reaction is monitored by HPLC using the HPLC method described in Example 10. A new peak with a retention time of 18.6 minutes appears and the peak corresponding to 5'-BCN-T₆-oligo at 18.1 minutes disappears. The reaction is complete in 75 minutes. The resulting solution of **37** is frozen and lyophilized.

| | |
|---|---|
| Calculated molecular weight: | 2,521.0 |
| Observed by mass spectrometry: | 2,521.2 |

### Example 30: Cu-free click conjugation of Methoxatin-TEG-Azide and 5'-BCN-T₆-oligo to provide 38.

A solution composed of 5'-BCN-T₆-oligo (-90 nmol), Methoxatin-TEG-azide (Berry & Associates, Inc. 180 nMol), 0.1 M aqueous triethylammonium acetate (pH 7, 0.45 mL) and acetonitrile (0.05 mL) is allowed to stand at room temperature. The progress of the Cu-free click reaction is monitored by HPLC using the HPLC method described in Example 16. A new peak with a retention time of 14.9 minutes appears and the peak corresponding to 5'-BCN-T₆-oligo at 18.1 minutes disappears. The reaction is complete in 36 hours. The resulting solution of **38** is frozen and lyophilized.

| | |
|---|---|
| Calculated molecular weight: | 2,653.0 |
| Observed by mass spectrometry: | 2,653.4 |

### Example 31: Cu-free click conjugation of Folate-TEG-Azide and 5'-BCN-T₆-oligo to provide 39.

A solution composed of 5'-BCN-T₆-oligo (-90 nmol), Folate-TEG-azide (Berry & Associates, Inc. catalog no. FC 8150, 180 nMol), 0.1 M aqueous triethylammonium acetate (pH 7, 0.45 mL) and acetonitrile (0.05 mL) is allowed to stand at room temperature. The progress of the Cu-free click reaction is monitored by HPLC using the HPLC method described in Example 10. A new peak with a retention time of 15.9 minutes appears and the peak corresponding to 5'-BCN-T₆-oligo at 18.1 minutes disappears. The reaction is complete in 12 hours. The resulting solution of **39** is frozen and lyophilized.

| | |
|---|---|
| Calculated molecular weight: | 2,748.1 |
| Observed by mass spectrometry: | 2,747.5 |

### Example 32: 5'-BCN-T₆-oligo with a short spacer. Substitution of 11 (example 2) for 10 in the method of Example 26 provides the analogous 5'-BCN-T₆-oligo with a shorter spacer between the BCN tag and the oligo.

| | |
|---|---|
| Calculated molecular weight: | 1988.4 |
| Observed by mass spectrometry: | 1988.6 |

## Claims

1. A compound of Formula **I:** 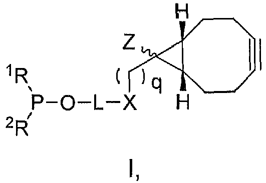 wherein:
q is 1, 2, or 3;
R¹- and R²- are independently N≡CCH₂CH₂O-, (C₁-C₆ alkyl)O-, or (C₁-C₆ alkyl)₂N-;
Z- is H-, or DMT-OCH₂-;
-X- and -L- are either both absent or both present;
-X- is absent or is -O-, -NH-, -S-, -NHCO₂-, -O₂CNH-, -NHCONH-, -NHCSNH-, or-CONH-; and
-L- is absent or is selected from a group consisting of -(CH₂)ₙ-, -(CH₂CH₂O)ₙ(CH₂)ₘ-, -(CH₂CH₂CH₂O)ₙ(CH₂)ₘ-, -(CH₂)₃S₂(CH₂)₃-, -(CH₂)₆S₂(CH₂)₆-,-(CH₂)₂O(CH₂)₃S₂(CH₂)₃O(CH₂)₂-, -CH(CH₂O-DMT)CH₂-, -CH(CH₂O-DMT)CH₂O(CH₂)ₘ-, -CH(CH₂CH₂O-DMT)CH₂CH₂-, -CH(CH₂CH₂O-DMT)CH₂CH₂O(CH₂)ₘ-, 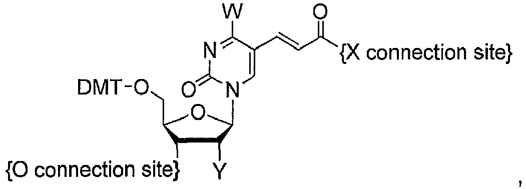 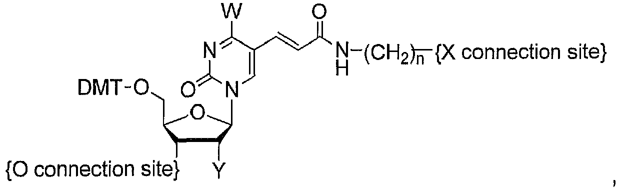 and 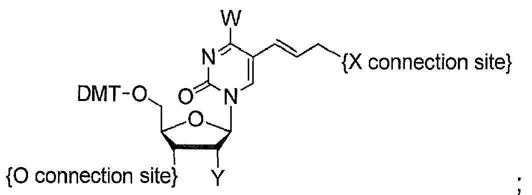
wherein n is 2-6, m is 2-3, Y is H, O-TBS, O-POM, or O-TOM, and W is OH, N=CHN(CH₃)₂, NHCOPh, or NHCOCH₃.

2. A compound according to claim 1, wherein R¹- is N≡CCH₂CH₂O- and R²- is (*i-*Pr)₂N.

3. A compound according to claim 1, wherein Z- is H- and -X- is -O-, -NH-, or-NHCO₂- or is absent.

4. A compound according to claim 1, wherein -L- is -(CH₂)ₙ-,-(CH₂CH₂O)ₙ(CH₂)ₘ-, or -(CH₂CH₂CH₂O)ₙ(CH₂)ₘ- or is absent.

5. A compound according to claim 1, wherein -L- is -(CH₂)₃S₂(CH₂)₃-,-(CH₂)₆S₂(CH₂)₆-, or -(CH₂)₂O(CH₂)₃S₂(CH₂)₃O(CH₂)₂-.

6. A compound according to claim 1, wherein -L- is

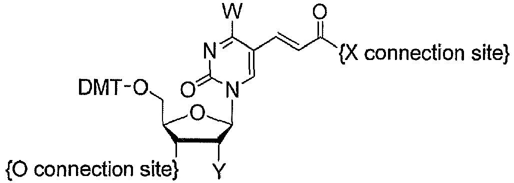

7. A compound according to claim 1, wherein -L- is

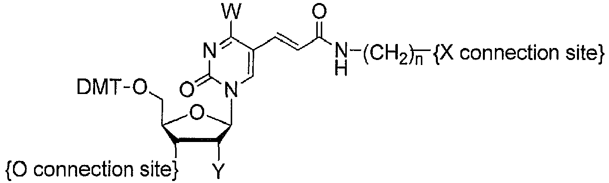

8. A compound according to claim 1, wherein -L- is

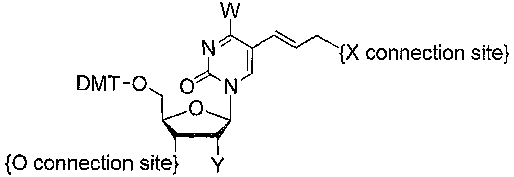

9. A compound according to any one of claims 1-8, wherein -X- and -L- are both present when q is 1.

10. A compound according to any one of claims 1-9, wherein q is 1.

11. A compound according to any one of claims 1-9, wherein q is 2.

12. A compound according to any one of claims 1-9, wherein q is 3.

13. A compound according to claim 1, wherein the compound is selected from a group consisting of: 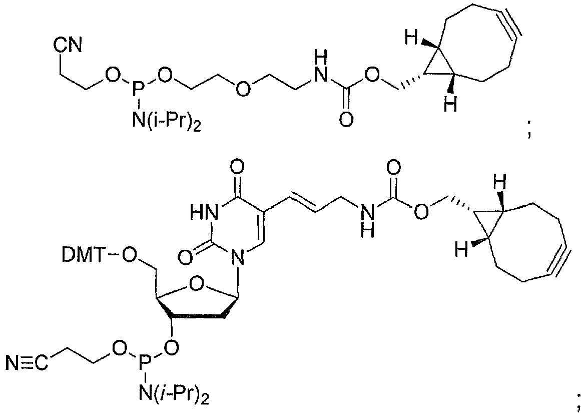 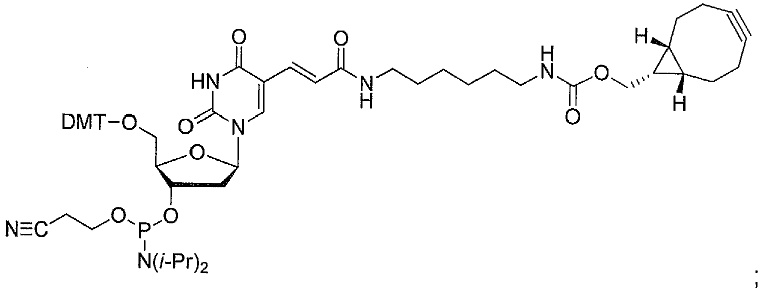 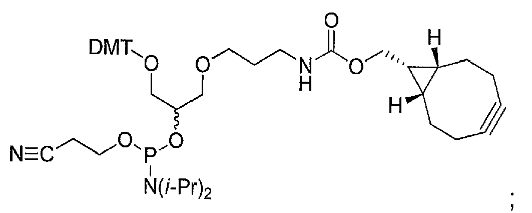 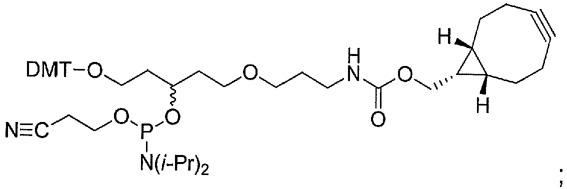 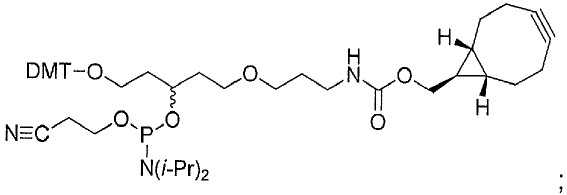 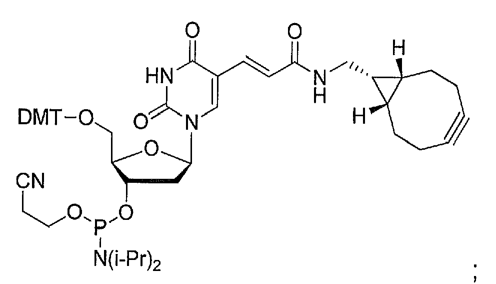 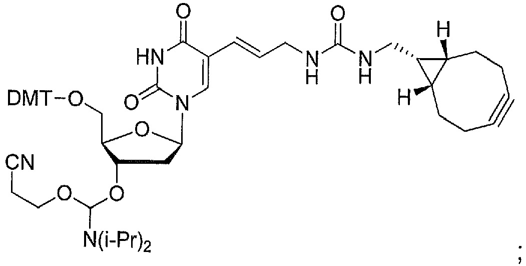 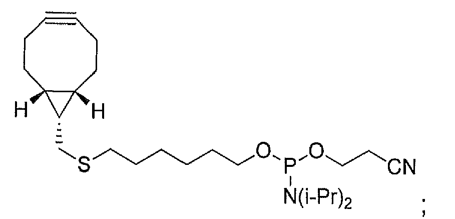 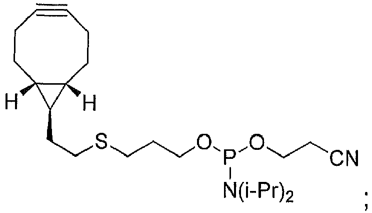 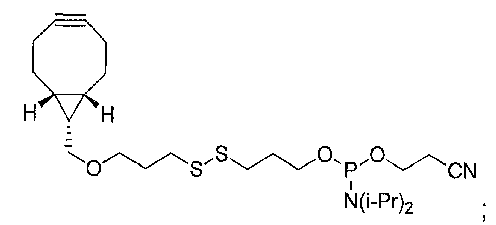 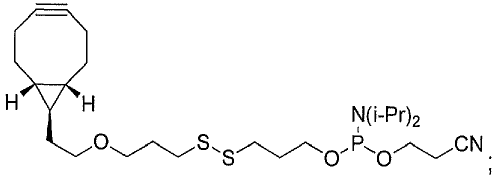 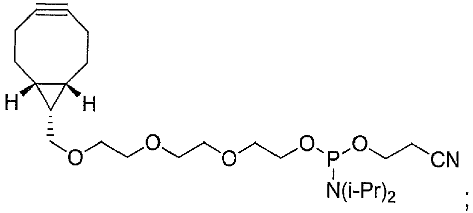 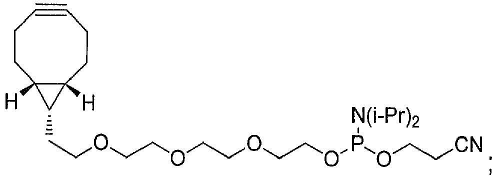 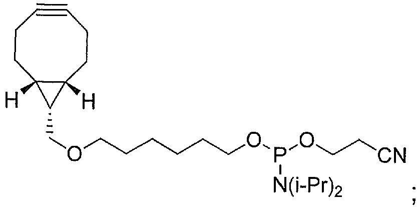 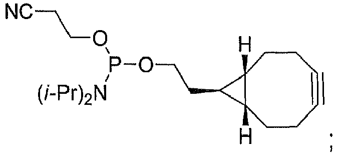 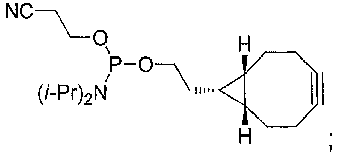 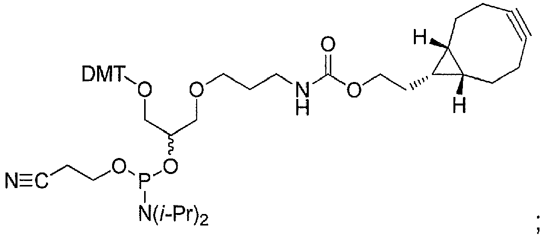 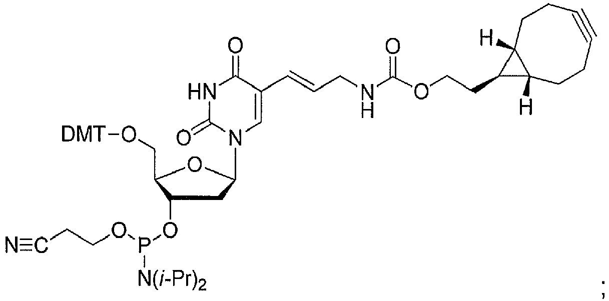 and

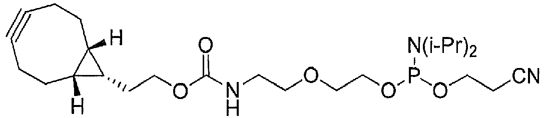

14. The compound according to claim 1 that is

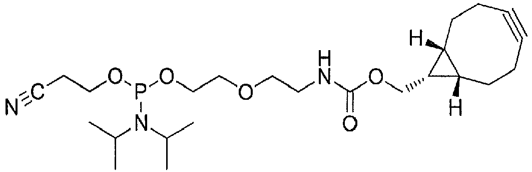

15. The compound according to claim 1 that is

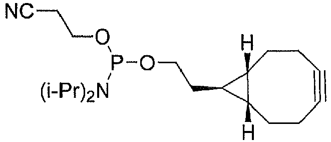

## Patentansprüche

1. Verbindung der Formel I: 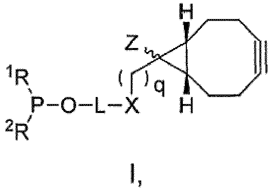 wobei:
q 1, 2 oder 3 ist;
R¹- und R²- unabhängig N≡CCH₂CH₂O-, (C₁-C₆Alkyl) O- oder (C₁-C₆Alkyl)₂N- sind;
Z- H- oder DMT-OCH₂- ist;
-X- und -L- entweder beide nicht vorhanden oder beide vorhanden sind;
-X- nicht vorhanden ist oder -O-, -NH-, -S-, -NHCO₂-, -O₂CNH-, -NHCONH-, -NHCSNH- oder -CONHist; und
-L- nicht vorhanden ist oder ausgewählt ist aus der Gruppe bestehend aus -(CH₂)ₙ-, - (CH₂CH₂O)ₙ(CH₂)ₘ-, - (CH₂CH₂CH₂O)ₙ(CH₂)ₘ-, - (CH₂)₃S₂(CH₂)₃-, - (CH₂)₆S₂(CH₂)₆-, - (CH₂)₂O(CH₂)₃S₂(CH₂)₃O(CH₂)₂-, -CH(CH₂O-DMT)CH₂-, -CH(CH₂O-DMT)CH₂O(CH₂)ₘ-, -CH(CH₂CH₂O-DMT)CH₂CH₂-, -CH(CH₂CH₂O-DMT)CH₂CH₂O(CH₂)ₘ-, 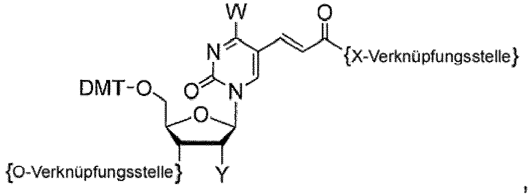 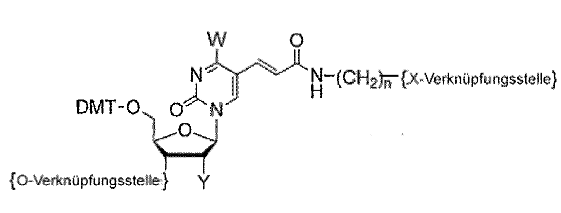 und 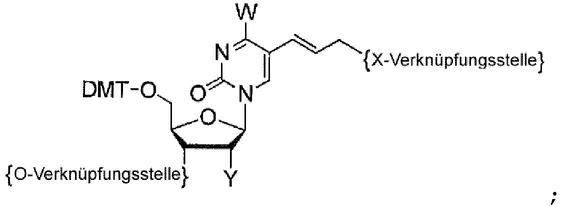
wobei n 2-6 ist, m 2-3 ist, Y H, O-TBS, O-POM oder O-TOM ist, und W OH, N=CHN(CH₃)₂, NHCOPh oder NHCOCH₃ ist.

2. Verbindung gemäß Anspruch 1, wobei R¹- N≡CCH₂CH₂O-ist und R²- (*i*-Pr)₂N ist.

3. Verbindung gemäß Anspruch 1, wobei Z- H- ist und -X- -0-, -NH- oder -NHCO₂ ist oder nicht vorhanden ist.

4. Verbindung gemäß Anspruch 1, wobei -L- -(CH₂)ₙ-, -(CH₂CH₂O)ₙ(CH2)ₘ- oder -(CH₂CH₂CH₂O)ₙ(CH₂)ₘ- ist oder nicht vorhanden ist.

5. Verbindung gemäß Anspruch 1, wobei -L- - (CH₂)₃S₂(CH₂)₃-, - (CH₂)₆S₂(CH₂)₆-, - (CH₂)₂O(CH₂)₃S₂(CH₂)₃O(CH₂)₂- ist.

6. Verbindung gemäß Anspruch 1, wobei -L- 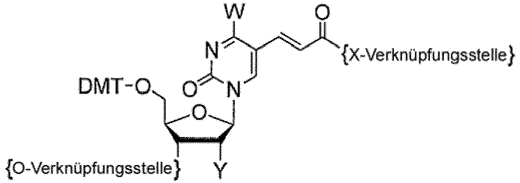 ist.

7. Verbindung gemäß Anspruch 1, wobei -L- 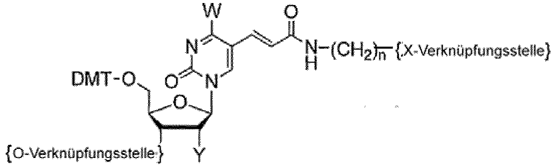 ist.

8. Verbindung gemäß Anspruch 1, wobei -L- 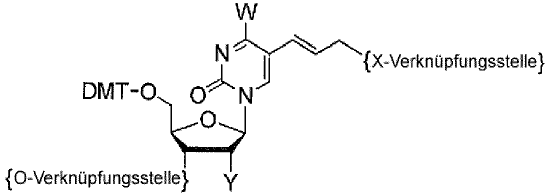 ist.

9. Verbindung gemäß einem der Ansprüche 1-8, wobei -X- und -L- beide vorhanden sind, wenn q 1 ist.

10. Verbindung gemäß einem der Ansprüche 1-9, wobei q 1 ist.

11. Verbindung gemäß einem der Ansprüche 1-9, wobei q 2 ist.

12. Verbindung gemäß einem der Ansprüche 1-9, wobei q 3 ist.

13. Verbindung gemäß Anspruch 1, wobei die Verbindung ausgewählt ist aus der Gruppe bestehend aus: 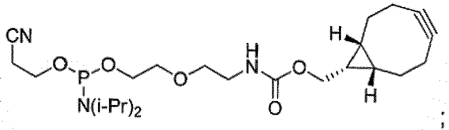 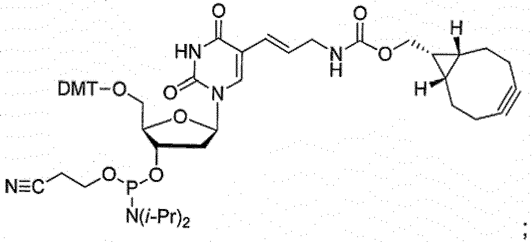 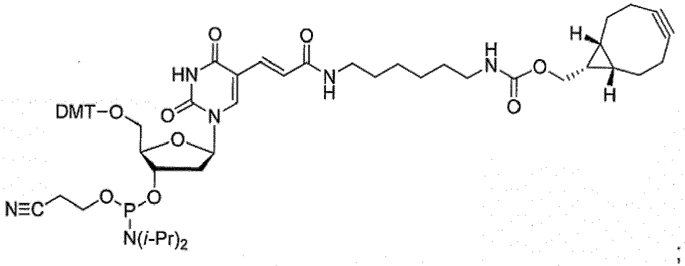 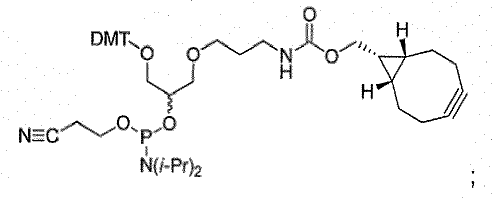 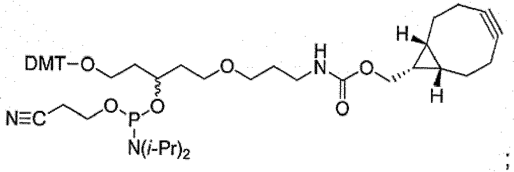 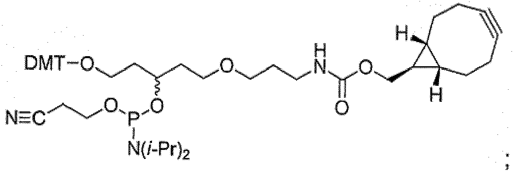 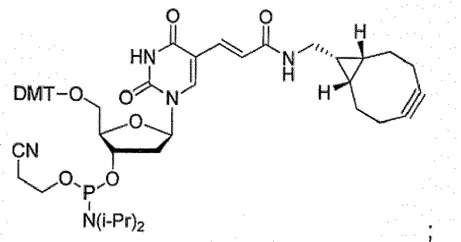 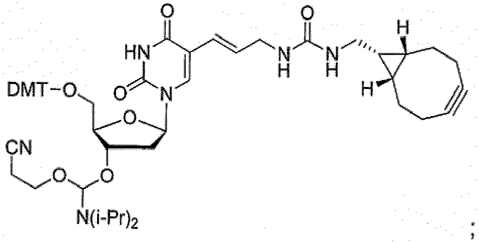 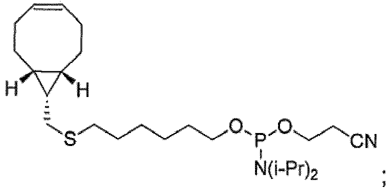 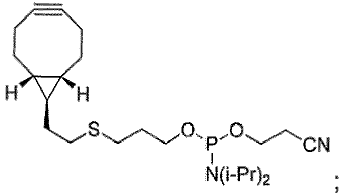 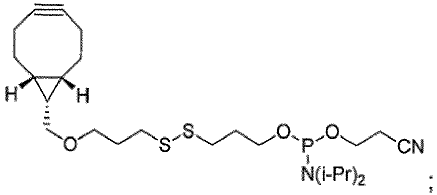 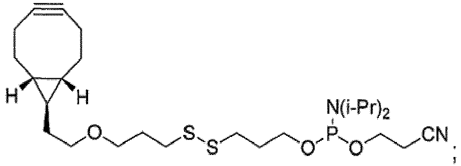 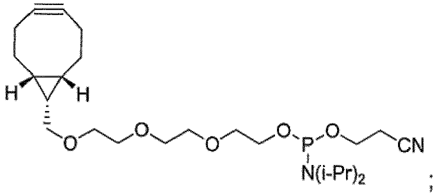 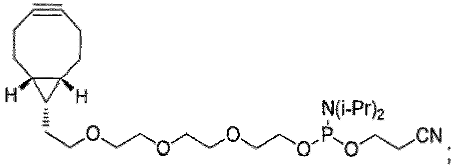 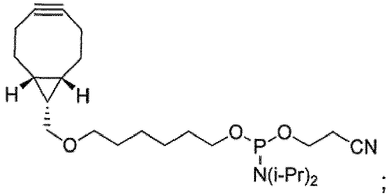 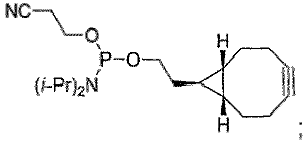 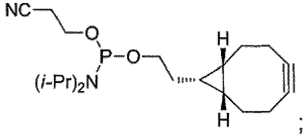 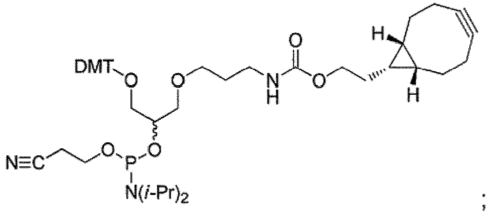 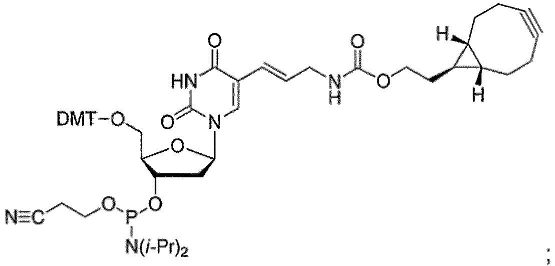 und

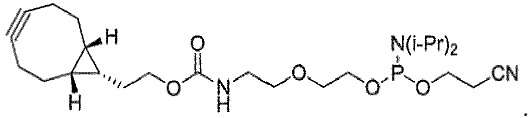

14. Verbindung gemäß Anspruch 1, die 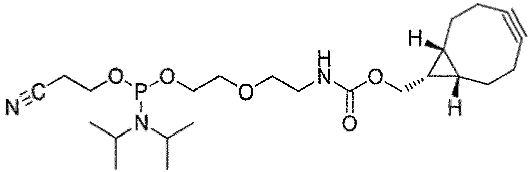 ist.

15. Verbindung gemäß Anspruch 1, die 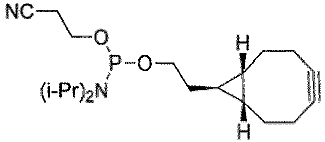 ist.

## Revendications

1. Composé de formule I : 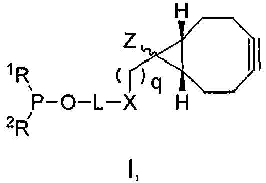 dans lequel :
q est 1, 2 ou 3 ;
R¹- et R²- sont indépendamment N=CCH₂CH₂O-, (alkyle en C₁-C₆,)O-, ou (alkyle en C₁-C₆)₂N- ;
Z- est H- ou DMT-OCH₂- ;
-X- et -L- sont tous deux absents ou tous deux présents ;
-X- est absent ou est -0-, -NH-, -S-, -NHCO₂-, -O₂CNH-, -NHCONH-, -NHCSNH-, ou -CONH- ; et
-L- est absent ou est choisi dans le groupe constitué de -(CH₂)ₙ-, - (CH₂CH₂O)ₙ(CH₂)ₘ-, -(CH₂CH₂CH₂O)ₙ(CH₂)ₘ-, - (CH₂)₃S₂(CH₂)₃-, -(CH₂)₆S₂(CH₂)₆-, - (CH₂)₂O(CH₂)₃S₂(CH₂)₃O(CH₂)₂-, -CH(CH₂O-DMT)CH₂-, -CH(CH₂O-DMT)CH₂O(CH₂)ₘ-, -CH(CH₂CH₂O-DMT)CH₂CH₂-, -CH(CH₂CH₂O-DMT)CH₂CH₂O(CH₂)ₘ-, 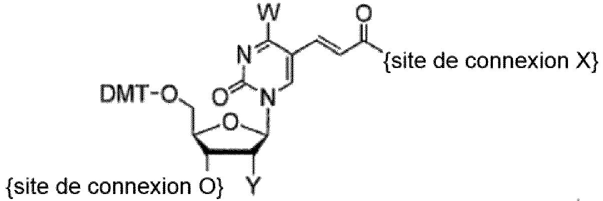 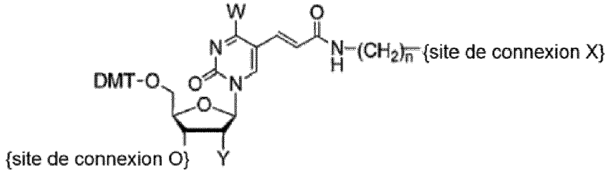 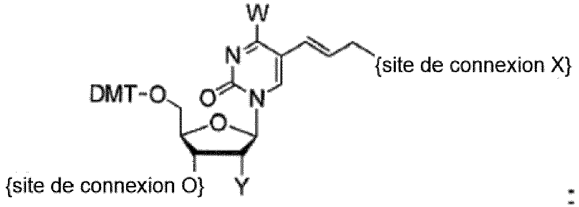
dans lequel n est 2 à 6, m est 2 à 3, Y est H, 0-TBS, O-POM, ou O-TOM, et W est OH, N=CHN(CH₃)₂, NHCOPh, ou NHCOCH₃.

2. Composé selon la revendication 1, dans lequel R¹- est N=CCH₂CH₂O- et R²- est (*i*-Pr)₂N.

3. Composé selon la revendication 1, dans lequel Z- est H- et -X- est -0-, -NH-, ou - NHCO₂- ou est absent.

4. Composé selon la revendication 1, dans lequel -L- est -(CH₂)ₙ-, -(CH₂CH₂O)ₙ(CH₂)ₘ-, ou -(CH₂CH₂CH₂O)ₙ(CH₂)ₘ- ou est absent.

5. Composé selon la revendication 1, dans lequel -L- est -(CH₂)₃S₂(CH₂)₃-, -(CH₂)₆S₂(CH₂)₆-, ou - (CH₂)₂O(CH₂)₃S₂(CH₂)₃O(CH₂)₂-.

6. Composé selon la revendication 1, dans lequel -L- est

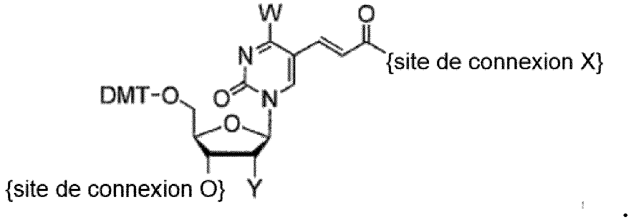

7. Composé selon la revendication 1, dans lequel -L- est

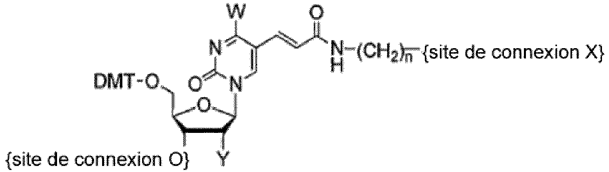

8. Composé selon la revendication 1, dans lequel -L- est

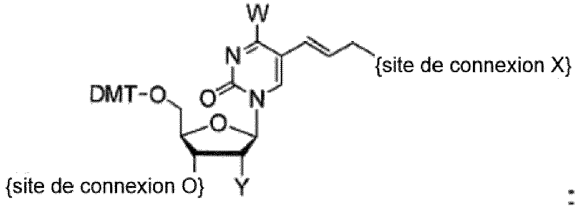

9. Composé selon l'une quelconque des revendications 1 à 8, dans lequel -X- et -L- sont tous deux présents lorsque q est 1.

10. Composé selon l'une quelconque des revendications 1 à 9, dans lequel q est 1.

11. Composé selon l'une quelconque des revendications 1 à 9, dans lequel q est 2.

12. Composé selon l'une quelconque des revendications 1 à 9, dans lequel q est 3.

13. Composé selon la revendication 1, **caractérisé en ce que** le composé est choisi dans le groupe constitué de : 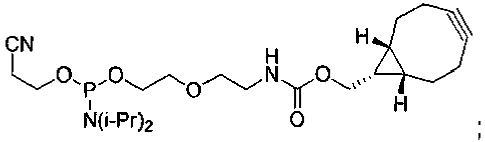 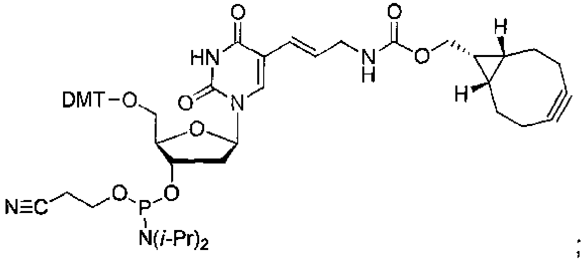 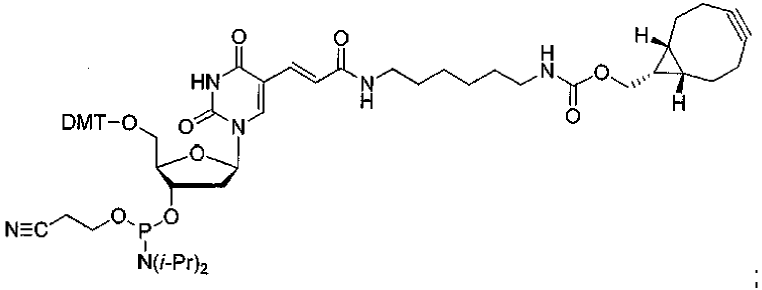 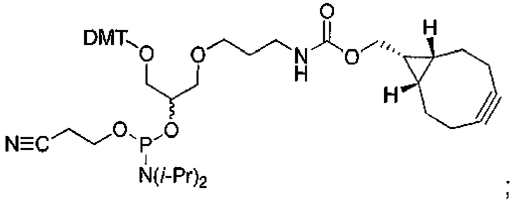 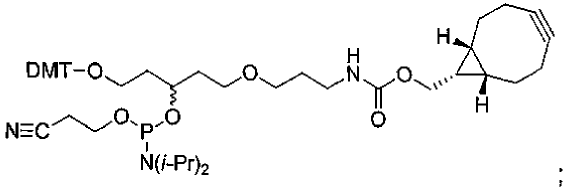 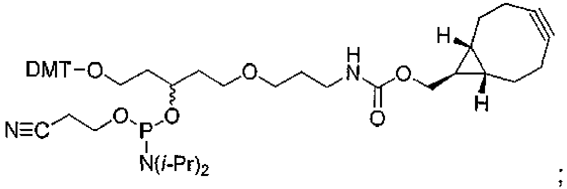 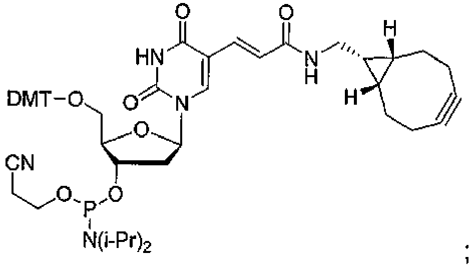 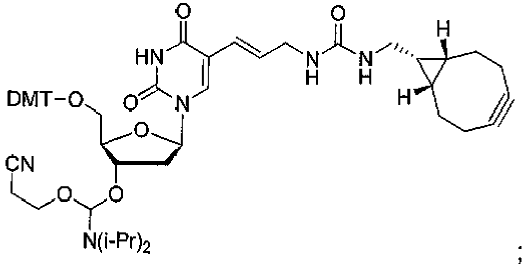 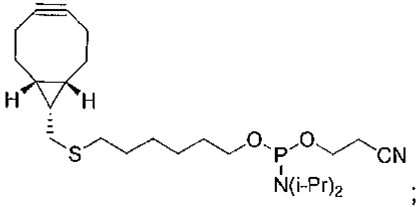 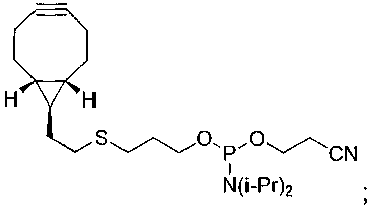 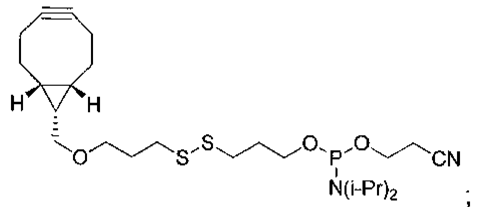 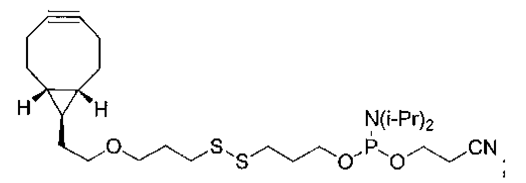 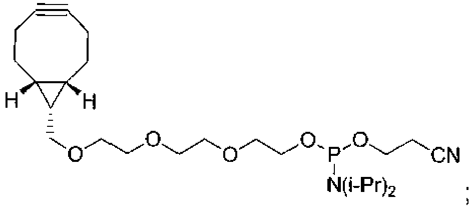 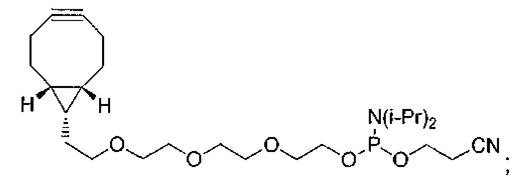 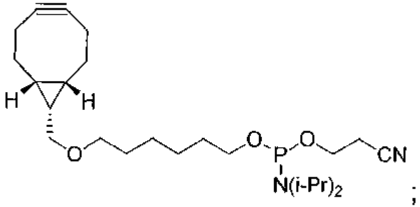 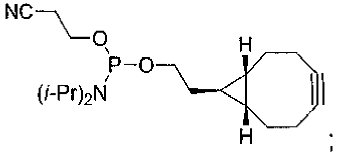 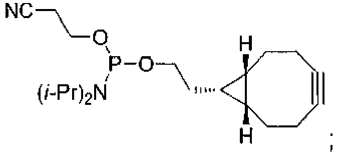 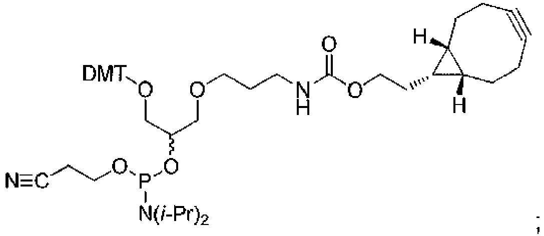 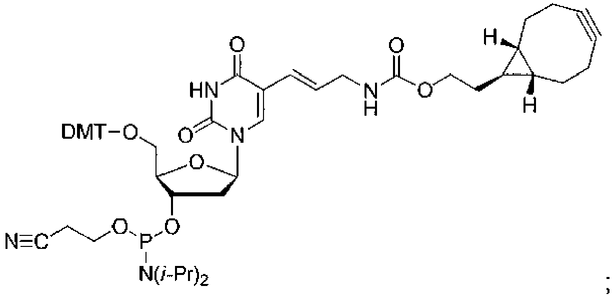 et

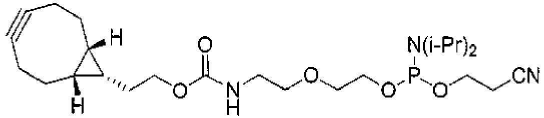

14. Composé selon la revendication 1 qui est

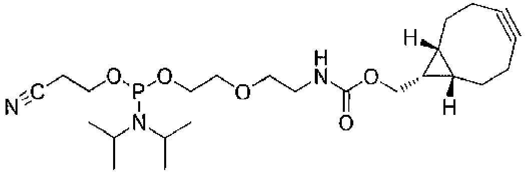

15. Composé selon la revendication 1 qui est
